# EUROPÄISCHE PATENTANMELDUNG

(11) **EP 4 830 674 A1**
(43) Veröffentlichungstag der Anmeldung: **28.10.2026**
(21) Anmeldenummer: 26173568.2
(22) Anmeldetag: 21.04.2026
(51) Int. Cl.: A61F 5/01, A61F 2/00, A61F 5/10, A61F 5/048, A61F 5/058

(54) **HANDORTHESE ZUR TRAKTION EINES FINGERGELENKS**

(30) Priorität: 25.04.2025 DE 102025116098
(71) Anmelder: Bort GmbH, 71384 Weinstadt-Benzach (DE)
(72) Erfinder: Freihaut, Roman, 73614 Schorndorf (DE); Bachmayer, Nicolaus, 70619 Stuttgart (DE)
(74) Vertreter: Thum, Bernhard

(57) **Zusammenfassung**

Die vorliegende Erfindung betrifft eine Handorthese (10; 110; 210) zur Traktion eines Fingergelenks, insbesondere eines Daumensattelgelenks (25; 225), eines Patienten, umfassend
einen ersten Orthesenabschnitt (14; 114; 214) zum Anbringen an einem Mittelhandbereich (16; 116; 216) einer Hand (12; 112; 212) des Patienten, wobei der erste Orthesenabschnitt (14; 114; 214) zumindest eine erste Öffnung (26; 126; 226) zum Einführen eines Fingers der Hand (12; 112; 212) des Patienten umfasst;
einen zweiten Orthesenabschnitt (18; 118; 218) zum Anbringen an dem Finger der Hand (12; 112; 212) des Patienten; und
zumindest eine Schienenanordnung (20, 22; 120; 220; 320; 420), die den ersten Orthesenabschnitt (14; 114; 214) und den zweiten Orthesenabschnitt (18; 118; 218) aneinanderkoppelt;
wobei die Schienenanordnung (20, 22; 120; 220; 320; 420) dazu vorgesehen ist, den zweiten Orthesenabschnitt (18; 118; 218) in einer vorgebbaren Lage relativ zu dem ersten Orthesenabschnitt (14; 114; 214) anzuordnen.

## Beschreibung

Die vorliegende Erfindung betrifft eine Handorthese zur Traktion eines Fingergelenks, insbesondere eines Daumensattelgelenks, eines Patienten.

Arthrose bei Fingergelenken ist eine weit verbreitete degenerative Erkrankung, die vor allem ältere Menschen betrifft und mit Schmerzen, Steifheit und eingeschränkter Beweglichkeit der betroffenen Gelenke einhergeht. Diese Beschwerden entstehen durch den fortschreitenden Abbau des Knorpelgewebes, was zu einer Reibung der Gelenkflächen und somit zu Entzündungen und weiteren Schmerzen führt. Insbesondere die Interphalangealgelenke (IPG) und das Daumensattelgelenk sind häufig betroffen, was zu einer erheblichen Beeinträchtigung der Lebensqualität führt.

Die Behandlung der Arthrose in den Fingergelenken umfasst in der Regel konservative Maßnahmen wie Schmerzmedikation, physikalische Therapie und in einigen Fällen auch orthopädische Hilfsmittel wie Handorthesen. Während diese Hilfsmittel in der Regel dazu dienen, die Gelenke zu stabilisieren und die Belastung zu reduzieren, ist die gezielte Schmerzlinderung ein zentraler Aspekt der Therapie.

Eine vielversprechende Methode zur Linderung der Beschwerden bei Arthrose ist das Ausüben einer Traktion auf das betroffene Gelenk. Es hat sich gezeigt, dass durch die Anwendung von Zugkräften eine Entlastung der Gelenkflächen erreicht werden kann, die den Schmerz verringert und die Funktion des Gelenks verbessert. Ferner hat sich eine manuelle Traktion in der Physiotherapie als eine konservative Therapiemöglichkeit etabliert. Die Traktion bewirkt eine Dehnung der Gelenkkapsel und des umgebenden Gewebes, wodurch der Druck auf die geschädigten Knorpelstrukturen verringert wird. Darüber hinaus kann eine gezielte Traktion den Stoffwechsel im betroffenen Gelenk stimulieren und so die Regeneration der Gelenkstrukturen fördern. Durch die richtige Anwendung einer Traktion in Kombination mit einer unterstützenden Orthese können Patienten mit Arthrose der Fingergelenke eine signifikante Schmerzlinderung und eine Verbesserung der Gelenkfunktion erfahren. Diese Methode ist besonders vorteilhaft, da sie eine nichtinvasive und konservative Behandlungsoption darstellt, die das Risiko von Nebenwirkungen im Vergleich zu chirurgischen Eingriffen oder medikamentösen Therapien reduziert.

Aus EP 3 280 356 B1 ist eine Orthese bekannt, bei der eine Grundkomponente und ein Ankerelement vorgesehen sind, wobei eine drehbar an der Grundkomponente angeordnete Spiralfeder das Ankerelement mit der Grundkomponente koppelt. Die Spiralfeder ist dazu vorgesehen ist, einen Daumen eines Patienten zu umgreifen. Die nahezu freie Bewegbarkeit des Daumens kann jedoch auch nachteilig sein. Beispielsweise wird eine Rotation des Daumensattelgelenks zugelassen. Eine solche Rotation kann jedoch Arthrose fördernd wirken. Zudem ist eine Oppositionsbewegung des Daumens relativ zu den anderen Fingern weitgehend zugelassen, obwohl diese Bewegung Arthrose fördernd wirkt.

Aus EP 3 280 356 B1 ist eine weitere Daumenorthese bekannt, bei der eine Grundkomponente an einem Unterarm eines Patienten angebracht ist und das Handgelenk überragt. Eine Schiene, deren Position relativ zu der Grundkomponente variabel ist, koppelt eine an dem Daumen angebracht Manschette mit der Grundkomponente. Ein Nachteil dieser Lösung ist, dass die Daumenorthese die Bewegung des Handgelenks stark einschränkt und damit der Tragekomfort für einen Patienten beeinträchtigt ist.

Vor dem Hintergrund ist es eine Aufgabe der Erfindung, eine Handorthese bereitzustellen, die speziell darauf ausgerichtet ist, eine Traktion auf das betroffene Fingergelenk auszuüben und gleichzeitig zumindest einen der erläuterten Nachteile des Stands der Technik zu überwinden.

Diese Aufgabe wird durch eine Handorthese zur Traktion eines Fingergelenks, insbesondere eines Daumensattelgelenks, eines Patienten gemäß Anspruch 1 gelöst. Die abhängigen Ansprüche betreffen vorteilhafte Weiterbildungen der Erfindung.

Ein Aspekt der Erfindung betrifft eine Handorthese zur Traktion eines Fingergelenks eines Patienten, umfassend
einen ersten Orthesenabschnitt zum Anbringen an einem Mittelhandbereich einer Hand des Patienten, wobei der erste Orthesenabschnitt zumindest eine erste Öffnung zum Einführen und/oder Aufnehmen eines Fingers der Hand des Patienten umfasst;
einen zweiten Orthesenabschnitt zum Anbringen an dem Finger der Hand des Patienten; und
zumindest eine Schienenanordnung, die den ersten Orthesenabschnitt und den zweiten Orthesenabschnitt aneinanderkoppelt;
wobei die Schienenanordnung dazu vorgesehen ist, den zweiten Orthesenabschnitt in einer vorgebbaren Lage relativ zu dem ersten Orthesenabschnitt anzuordnen.

Bevorzugt betrifft das Fingergelenk ein Daumengelenks, insbesondere ein Daumensattelgelenk. Entsprechend ist die erste Öffnung vorzugsweise zum Einführen bzw. Aufnehmen des Daumens der Hand des Patienten vorgesehen. Ferner ist der zweite Orthesenabschnitt zum Anbringen an dem Daumen der Hand des Patienten vorgesehen.

Die Schienenanordnung erlaubt es dem Patienten oder einem Therapeuten, die Lage des zweiten Orthesenabschnitts relativ zu dem ersten Orthesenabschnitt bedarfsabhängig vorzugeben, um so das Ausmaß an Traktion auf das Fingergelenk anzupassen. Daneben bietet die Schienenanordnung eine zusätzliche Festigkeit für die Handorthese, wobei die Bewegbarkeit des Daumens eingeschränkt wird, um Arthrose fördernde Belastungen wie beispielsweise eine Torsion oder übermäßige Beugung des Daumensattelgelenks zu vermeiden. Ferner kann aufgrund der Schienenanordnung eine Abduktions- und Adduktionsbewegung insbesondere im Falle eines Daumens gezielt eingeschränkt und/oder vorgegeben werden, um Arthrose fördernde Belastungen zu vermeiden.

Ein weiterer Vorteil der erfindungsgemäßen Handorthese ist ihre schlanke Ausbildung. Dadurch, dass der erste Orthesenabschnitt zum Anbringen an dem Mittelhandbereich der Hand des Patienten ausgebildet ist und der zweite Orthesenabschnitt zum Anbringen an dem Finger, wird die Bewegung des Arms, des Handgelenks und/oder der Hand, abgesehen von dem Finger, an dem der zweite Orthesenabschnitt angebracht ist, nicht oder kaum eingeschränkt.

Gemäß einem Aspekt der Schienenanordnung kann vorgesehen sein, dass die Schienenanordnung
- zumindest ein erstes längliches Schienenelement, das mit zumindest einem von dem ersten und dem zweiten Orthesenabschnitt gekoppelt ist; und
- eine betätigbare Verstellanordnung umfasst;
wobei die Verstellanordnung dazu eingerichtet ist, bei Betätigung ein Ändern, insbesondere translatorisches Ändern, der Lage des ersten länglichen Schienenelements und dem damit gekoppelten Orthesenabschnitt relativ zu dem anderen von dem ersten und dem zweiten Orthesenabschnitt zuzulassen.

Das erste längliche Schienenelement verleiht der Schienenanordnung und damit der Handorthese zusätzliche Stabilität. Ferner ermöglicht die Verstellanordnung, die Lage des zweiten Orthesenabschnitts je nach gewünschter Traktion auf das Fingergelenk, insbesondere das Daumensattelgelenk, einzustellen und damit die Lage des zweiten Orthesenabschnitts relativ zu dem ersten Orhesenabschnitt vorgebbar auszubilden. Vereinfacht gesagt ermöglicht die Verstellanordnung ein Anpassen und Vorgeben der Traktion auf das Fingergelenk. Es versteht sich, dass der erste Orthesenabschnitt an dem Mittelhandbereich und der zweite Orthesenabschnitt an dem Finger anbringbar sind, damit die Traktion auf das Fingergelenk auftreten kann. Zudem ermöglicht die Verstellanordnung, dass das Ausmaß der Traktion durch Ändern der Lage angepasst und entsprechend vorgegeben werden kann, wenn die Handorthese an der Hand angelegt ist, sowie zumindest eingeschränkt auch dann, wenn die Handorthese nicht an der Hand angelegt ist, nämlich indem die Handorthese für das Tragen an einer Hand mit einer beabsichtigten Traktion vorab eingestellt werden kann. Daneben ermöglicht die Verstellanordnung, eine Abduktions- und Adduktionsbewegung insbesondere im Falle eines Daumens gezielt einzuschränken und/oder vorzugeben, um Arthrose fördernde Belastungen zu vermeiden.

Dadurch, dass das erste längliche Schienenelement mit einem der Orthesenabschnitte gekoppelt ist, führt das Ändern der Lage des ersten länglichen Schienenelements relativ zu dem anderen von dem ersten und zweiten Orthesenabschnitt auch zu einer Veränderung der Lage des Orthesenabschnitts relativ zu dem anderen Orthesenabschnitt. Ist das erste längliche Schienenelement also beispielsweise mit dem zweiten Orthesenabschnitt gekoppelt, so führt eine Veränderung der Lage des ersten länglichen Schienenelements relativ zu dem ersten Orthesenabschnitt auch zu einer Veränderung der Lage des zweiten Orthesenabschnitts relativ zu dem ersten Orthesenabschnitt. Die entsprechende Änderung der Lage kann dabei gleich sein. Durch das gezielte Ändern der Lage des ersten länglichen Schienenelements relativ zu dem anderen von dem ersten und zweiten Orthesenabschnitt ist die Lage des zweiten Orthesenabschnitts relativ zu dem ersten Orthesenabschnitt vorgebbar.

Wenn zwei Gegenstände miteinander gekoppelt sind, dann kann dies umfassen, dass die beiden Gegenstände bevorzugt direkt aneinander angebracht sind und/oder eine Bewegung des einen Gegenstands zu einer gleichen Bewegung des anderen Gegenstands führt. Die Handorthese ist dann an der Hand angebracht, wenn der erste Orthesenabschnitt an dem Mittelhandbereich der Hand bevorzugt positionstreu und der zweite Orthesenabschnitt dem Finger der Hand des Patienten bevorzugt positionstreu anliegt. Ein Betätigen der Verstellanordnung kann beispielsweise ein Drücken und/oder ein Drehen der Verstellanordnung umfassen.

Gemäß einer Ausgestaltung der Erfindung ist die Verstellanordnung dazu vorgesehen, bei Betätigung der Verstellanordnung eine Änderung der Lage mittels diskreter Positionen zuzulassen. So ist die Lage anhand diskreter Positionen vorgebbar. Die diskreten Positionen ermöglichen ein vereinfachtes Einstellen der Handorthese. Ferner erleichtert das Vorsehen der diskreten Positionen dem Patienten oder Therapeuten, eine derzeit eingestellte Position zu identifizieren und/oder eine gewünschte Position einzustellen, um eine gewünschte Lage vorzugeben. Ferner ermöglichen es die diskreten Positionen einem Patienten, eine von einem Therapeuten vorgeschlagene Lage des zweiten Orthesenabschnitts relativ zu dem ersten Orthesenabschnitt zuverlässig zu reproduzieren. So kann eine vom Therapeuten gewünschte Traktion auf das Gelenk einfach reproduziert werden.

Gemäß einer Weiterbildung der Erfindung kann vorgesehen sein, dass die Verstellanordnung bei Nicht-Betätigung der Verstellanordnung eine Änderung der Lage zumindest in einer ersten Richtung zu sperren und bei Betätigung der Verstellanordnung eine Änderung der Lage zumindest in der ersten Richtung freizugeben. Dadurch kann die Verstellanordnung gezielt in zumindest zwei Zustände versetzt werden, und eine Veränderung der Lage ist nur bei Betätigung der Verstellanordnung möglich.

Gemäß einer Ausgestaltung der Erfindung kann vorgesehen sein, dass die Verstellanordnung ferner dazu eingerichtet ist, bei Nicht-Betätigung der Verstellanordnung eine Änderung der Lage in einer zweiten Richtung zu sperren und bei Betätigung der Verstellanordnung eine Änderung der Lage in der zweiten Richtung freizugeben. Vorzugsweise ist die zweite Richtung zu der ersten Richtung entgegengesetzt ausgerichtet. Dadurch kann die Verstellanordnung gezielt in zumindest zwei weitere Zustände versetzt werden, und eine Veränderung der Lage in der zweiten Richtung ist nur bei Betätigung der Verstellanordnung möglich.

Gemäß einer alternativen Ausgestaltung der Erfindung kann vorgesehen sein, dass die Verstellanordnung dazu eingerichtet ist, eine Änderung der Lage in einer zweiten Richtung sowohl bei einer Nicht-Betätigung als auch bei einer Betätigung der Verstellanordnung zuzulassen. Man denke in diesem Zusammenhang beispielsweise an einem Kabelbinder, der eine Betätigung in einer Richtung zulässt, um den Kabelbinder festzuziehen, während eine Betätigung in einer entgegengesetzten Richtung unterbunden ist, da ansonsten der Kabelbinder wieder geöffnet werden würde.

Vorzugsweise führt eine Änderung der Lage in der zweiten Richtung zu einer erhöhten Traktion des Fingergelenks, wohingegen eine Änderung der Lage in der ersten Richtung zu einer verringerten Traktion des Fingergelenks führen würde. Ist dann eine Änderung der Lage in der zweiten Richtung sowohl bei einer Nicht Betätigung als auch bei einer Betätigung der Verstellanordnung zugelassen, dann kann der Patient oder ein Therapeut die Traktion auf das Fingergelenk auf besonders einfache Weise erhöhen. Demgegenüber kann ein unbeabsichtigtes Verringern der Traktion auf das Fingergelenk vermieden werden, da die Verstellanordnung zuerst betätigt werden muss, um eine Änderung der Lage der ersten Richtung zuzulassen. Die zweite Richtung kann in Richtung eines distalen Endes des Daumens orientiert sein.

Erfindungsgemäß ist das erste längliche Schienenelement mit einem von dem ersten und dem zweiten Orthesenabschnitt gekoppelt. Es kann dann vorgesehen sein, dass die Verstellanordnung mit dem anderen von dem ersten und dem zweiten Orthesenabschnitt gekoppelt ist.

Gemäß einer Weiterbildung der Erfindung kann vorgesehen sein, dass die Verstellanordnung ein Schneckengetriebe umfasst, das dazu vorgesehen ist, bei Betätigung ein Ändern, insbesondere translatorisches Ändern, der Lage des ersten länglichen Schienenelements und dem damit gekoppelten Orthesenabschnitt relativ zu dem anderen von dem ersten und dem zweiten Orthesenabschnitt zu bewirken. Das Schneckengetriebe kann eine Schneckenwelle umfassen. In einer Ausführungsform kann vorgesehen sein, dass die Schneckenwelle dazu vorgesehen ist, bei Betätigung derselben ein Ändern, insbesondere translatorisches Ändern, der Lage des ersten länglichen Schienenelements und dem damit gekoppelten Orthesenabschnitt relativ zu dem anderen von dem ersten und dem zweiten Orthesenabschnitt zu bewirken. Die Schneckenwelle kann ein spiralförmig ausgebildetes Gewinde umfassen. Die Schneckenwelle kann dazu vorgesehen sein, in Zähne einzugreifen, die an dem ersten länglichen Schienenelement ausgebildet sind. Das Schneckengetriebe kann dazu vorgesehen sein, eine rotatorische Bewegung, insbesondere der Schneckenwelle um eine Schneckenwellenlängsachse, in eine translatorische Bewegung des zweiten Orthesenabschnitts relativ zu dem ersten Orthesenabschnitt, insbesondere des ersten länglichen Schienenelements relativ zu der Schneckenwelle und/oder dem ersten Orthesenabschnitt und/oder dem zweiten Orthesenabschnitt, zu übertragen. Ein Vorteil der Ausbildung der Verstellanordnung mit Schneckengetriebe ist, dass das Schneckengetriebe einfach durch einen Bediener betätigt werden kann. Dazu kann die Schneckenwelle beispielsweise mit einem Finger des Bedieners in einer der Schneckenwellendrehrichtungen gedreht werden. Ein weiterer Vorteil ist die selbsthemmende Eigenschaft des Schneckengetriebes. Dies bedeutet, dass das Gewinde, mit dem die Schneckenwelle in Eingriff ist, die Schneckenwelle nicht drehen kann. So kann ein Bediener auf einfache Weise durch Betätigen des Schneckengetriebes den zweiten Orthesenabschnitt in einer gewünschten Lage relativ zu dem ersten Orthesenabschnitt anordnen, wobei eine Kraft auf die Orthesenabschnitte nicht zu einer Betätigung der Verstellanordnung führt. Ein wiederum weiterer Vorteil ist die schlanke Ausführung der Schienenanordnung mit dem Schneckengetriebe.

Die Schneckenwelle kann eine raue, insbesondere geriffelte, Oberfläche umfassen. Eine solche raue Oberfläche kann in einem Bereich der Schneckenwelle vorgesehen sein, der zum Betätigen durch einen Bediener vorgesehen ist. Die raue Oberfläche führt zu einem verbesserten Kontakt eines Fingers des Bedieners mit der Schneckenwelle, sodass höhere Kräfte ohne Abrutschen durch den Finger übertragen werden können. Insgesamt verbessert die raue Oberfläche den Kontakt zwischen Finger und Schneckenwelle und damit die Betätigbarkeit der Schneckenwelle durch den Bediener.

Gemäß einer Ausgestaltung der Erfindung kann vorgesehen sein, dass die Verstellanordnung eine selbsthemmende Eigenschaft aufweist. Eine solche selbsthemmende Eigenschaft ist beispielsweise bei einem Schneckengetriebe ausgebildet.

Gemäß einer Weiterbildung der Erfindung kann vorgesehen sein, dass die Verstellanordnung zumindest bei Betätigen ein Gleiten des ersten länglichen Schienenelements und dem damit gekoppelten Orthesenabschnitt relativ zu dem anderen von dem ersten und dem zweiten Orthesenabschnitt zuzulassen.

Gemäß einer Weiterbildung der Erfindung kann vorgesehen sein, dass die Verstellanordnung zumindest bei Nicht-Betätigen ein Gleiten des ersten länglichen Schienenelements und dem damit gekoppelten Orthesenabschnitt relativ zu dem anderen von dem ersten und dem zweiten Orthesenabschnitt sperrt.

Gemäß einer Weiterbildung der Erfindung kann vorgesehen sein, dass die Verstellanordnung eine Klemmanordnung und/oder eine Schraubverbindung umfasst, die dazu vorgesehen ist, bei Betätigung ein Ändern, insbesondere translatorisches Ändern, der Lage des ersten länglichen Schienenelements und dem damit gekoppelten Orthesenabschnitt relativ zu dem anderen von dem ersten und dem zweiten Orthesenabschnitt zuzulassen. Dabei kann vorgesehen sein, dass die Klemmanordnung und/oder die Schraubverbindung bei Nicht-Betätigen ein Ändern der Lage des ersten länglichen Schienenelements und dem damit gekoppelten Orthesenabschnitt relativ zu dem anderen von dem ersten und dem zweiten Orthesenabschnitt zu sperren. Es kann vorgesehen sein, dass die Klemmanordnung und/oder die Schraubverbindung ein geometrisches Merkmal zum Greifen und Betätigen durch einen Benutzer umfasst. So kann die Verstellanordnung auf einfache Weise händisch betätigt werden.

Gemäß einer Weiterbildung der Erfindung ist das erste längliche Schienenelement Y-förmig ausgebildet. So kann das erste längliche Schienenelement einen länglichen Grundabschnitt und zumindest zwei mit dem länglichen Grundabschnitt gekoppelte Gabelabschnitte umfassen. Die Gabelabschnitte können im Wesentlichen spiegelbildlich zu einer Längsachse des Grundabschnitts ausgebildet sein. Vorzugsweise sind die Gabelabschnitte mit einem von dem ersten und dem zweiten Orthesenabschnitt gekoppelt, wohingegen der Grundabschnitt dem anderen von dem ersten und den zweiten Orthesenabschnitt zuzuordnen ist. In einer vorteilhaften Ausgestaltung sind die Gabelabschnitte mit dem zweiten Orthesenabschnitt gekoppelt, wobei die Verstellanordnung bei Betätigung ein Ändern der Lage der Verstellanordnung relativ zu dem ersten Orthesenabschnitt zulässt. Auf diese Weise kann die Kraft auf den zweiten Orthesenabschnitt zur Ausübung der Traktion über die mehreren Gabelabschnitte übertragen werden. Somit kann eine aufgeteilte Krafteinleitung auf den zweiten Orthesenabschnitt erreicht werden. Ferner ermöglicht die aufgeteilte Krafteinleitung eine gleichmäßigere Traktionsausübung auf das Fingergelenk. Der eine Grundabschnitt kann es ferner ermöglichen, für diese Schienenanordnung genau eine Verstellanordnung vorzusehen. Insgesamt kann so einfacher Aufbau der Handorthese erreicht werden. Zudem ist die Bedienung einfach, da beispielsweise nur die Lage des einen Grundabschnitts vorgegeben werden muss, um den zweiten Orthesenabschnitt relativ zu dem ersten Orthesenabschnitt anzuordnen. Gemäß einer Ausgestaltung ist das erste längliche Schienenelement eine einstückige längliche Schiene.

Es kann vorgesehen sein, dass die Gabelabschnitte eine Aussparung ausbilden, die dazu vorgesehen ist, an der Hand des Patienten an einem Gelenk der Hand angeordnet zu sein. Im Fall der Ausbildung der Handorthese als Daumenorthese kann die Aussparung beispielsweise dazu vorgesehen sein, an dem Daumengrundgelenk angeordnet zu sein. Dies ermöglicht einen angenehmen Tragekomfort und ein aufgrund der Aussparung nicht unangenehm wirkendes Anliegen an dem Daumengrundgelenk. Aufgrund von Arthrose können an dem Gelenkrand des Gelenks sogenannte Otheophyten, also knöcherne Auswüchse, als Reaktion auf Knorpelverschleiß ausgebildet sein, die eine Vergrößerung im Bereich des Gelenks darstellen. Ferner kann sich Flüssigkeit im Gelenk aufgrund einer Entzündungsreaktion ansammeln, was als Schwellung wahrgenommen werden kann. Die Aussparung kann dazu vorgesehen sein, den Bereich der Vergrößerung und/oder der Schwellung aufzunehmen, sodass auf diese in der Regel empfindlichen Bereiche kein oder weniger Druck ausgeübt wird oder dieser Bereich von der Handorthese nicht berührt wird.

Gemäß einer Weiterbildung der Erfindung kann vorgesehen sein, dass das längliche Schienenelement im Wesentlichen starr und/oder biegesteif und/oder torsionssteif ausgebildet ist. Ferner kann vorgesehen sein, dass das längliche Schienenelement starrer und/oder biegesteifer und/oder torsionssteifer ausgebildet ist als der erste und/oder der zweite Orthesenabschnitt.

Gemäß einer Weiterbildung der Erfindung kann vorgesehen sein, dass die Schienenanordnung ferner ein zweites längliches Schienenelement umfasst, das mit dem anderen von dem ersten und dem zweiten Orthesenabschnitt gekoppelt ist. Das zweite längliche Schienenelement kann wie vorausgehend beschrieben zum ersten länglichen Schienenelement ausgebildet sein. Gemäß einer Ausgestaltung ist das zweite längliche Schienenelement eine einstückige längliche Schiene.

Gemäß einer Ausgestaltung der Erfindung kann vorgesehen sein, dass die Verstellanordnung dazu eingerichtet ist, bei Betätigung ein Ändern, insbesondere translatorisches Ändern, der Lage des zweiten länglichen Schienenelements und dem damit gekoppelten Orthesenabschnitt relativ zu dem ersten länglichen Schienenelement zuzulassen, nämlich in der ersten und/oder der zweiten Richtung. So können zwei Schienenelemente bereitgestellt werden, die relativ zueinander bewegbar sind. Ferner kann die Verstellanordnung dazu eingerichtet sein, bei Nicht-Betätigung ein Ändern, insbesondere translatorisches Ändern, der Lage des zweiten länglichen Schienenelements und dem damit gekoppelten Orthesenabschnitt relativ zu dem ersten länglichen Schienenelement zu sperren, insbesondere in der ersten und/oder zweiten Richtung.

Die Verstellanordnung kann gemäß einer Weiterbildung relativ zu dem zweiten länglichen Schienenelement verschieblich gelagert angeordnet sein.

Das zweite längliche Schienenelement ist gemäß einer Ausgestaltung fest oder zumindest positionstreu an dem ersten und/oder zweiten Orthesenabschnitt angebracht.

Gemäß einer Weiterbildung der Erfindung kann vorgesehen sein, dass die Schienenanordnung im Wesentlichen starr und/oder biegesteif und/oder torsionssteif ausgebildet ist.

In einer bevorzugten Ausführungsform umfasst die Schienenanordnung, insbesondere eines der Schienenelemente, einen Anlageabschnitt zum Anliegen an einem Finger, insbesondere an dem Daumen, des Patienten. Bevorzugt betrifft der Anlageabschnitt ein Ende, insbesondere distales Ende, der Schienenanordnung, insbesondere des Schienenelements. Der Anlageabschnitt ist bevorzugt T-förmig ausgebildet, könnte aber auch eine andere Form aufweisen und beispielsweise D- oder O-förmig ausgebildet sein. Der Anlageabschnitt kann zumindest abschnittsweise und zumindest auf einer palmaren Seite des Anlageabschnitts der Form des Fingers, insbesondere des Daumens, nachempfunden sein. Die Form kann dabei die äußere Kontur des Fingers im Bereich des Fingerendgelenks, insbesondere des Daumenendgelenk, zumindest teilweise umfassen. Dadurch wird ein komfortables Anliegen der Schienenanordnung im Bereich des zweiten Orthesenabschnitts am Finger bewirkt. Ist der Anlageabschnitt zum Anliegen im Bereich der proximalen Kondylen des Daumenendgelenks vorgesehen, so kann vorgesehen sein, dass der Anlageabschnitt der Form der proximalen Kondylen des Daumenendgelenks zumindest teilweise nachempfunden ist. Der Anlageabschnitt kann der anatomischen Form der Kondylen nachempfunden sein. Man könnte sagen, dass der Anlageabschnitt bevorzugt unterhalb des Daumenendgelenks, besonders bevorzugt infrakondylär, eine anatomische Hintergreifung zum Anliegen des Anlageabschnitts ermöglicht. Der Anlageabschnitt kann zum Koppeln mit dem zweiten Orthesenabschnitt ausgebildet sein. Der Anlageabschnitt kann zur Aufnahme in einer Tasche und/oder Öffnung des zweiten Orthesenabschnitts vorgesehen sein.

Eine Weiterbildung der bevorzugten Ausführungsform sieht vor, dass die Schienenanordnung zumindest ein erstes Material und im Bereich des Anlageabschnitts ein zweites Material umfasst. Das zweite Material kann zumindest an einer palmaren Seite des Anlageabschnitts vorgesehen sein. Eine dorsale Seite des Anlageabschnitts kann frei von dem zweiten Material sein. Vorzugsweise ist das zweite Material weicher als das erste Material. Ferner kann das zweite Material auf der Schienenanordnung aufgebracht sein. Ein solches Aufbringen kann durch Beschichten, insbesondere Umspritzen, erfolgen. Das zweite Material kann thermoplastisches Polyurethan (TPU) umfassen. Das weichere zweite Material, insbesondere das TPU, ermöglicht eine gute Druckumverteilung und sorgt deshalb für ein angenehmes Anliegen der Schienenanordnung an dem Finger. Lokale Druckspitzen können reduziert oder gar vermieden werden. Sofern der Anlageabschnitt zum direkten Anliegen auf der Haut vorgesehen ist, sorgt das TPU ferner für einen guten, rutschfesten Kontakt mit dem Finger, insbesondere dem Daumen. Insgesamt kann vorgesehen sein, dass das erste Material eine höhere Steifigkeit als das zweite Material aufweist. Das erste Material kann thermoplastisches, insbesondere spritzgießfähiges Material umfassen. Dabei kann das erste Material Polypropylen, Polyethylen, Polycarbonat und/oder Polyamid umfassen.

Eine Weiterbildung der bevorzugten Ausführungsform sieht vor, dass die Schienenanordnung, insbesondere der Anlageabschnitt, einen Koppelabschnitt zum Koppeln mit dem zweiten Orthesenabschnitt umfasst. Der Koppelabschnitt kann an einer dorsalen Seite des Anlageabschnitts vorgesehen sein. Der Koppelabschnitt kann einen Vorsprung zur Aufnahme in einer Öffnung des zweiten Orthesenabschnitts umfassen. So kann der zweite Orthesenabschnitt einfach mit dem Orthesenabschnitt gekoppelt werden. Beispielsweise kann der zweite Orthesenabschnitt mittels Aufsteckens auf den Vorsprung mit diesem verbunden werden. Umfasst der zweite Orthesenabschnitt beispielsweise Stoffmaterial und/oder einen Klettverschluss, kann der zweite Orthesenabschnitt zunächst mit Koppelabschnitt, insbesondere dem Vorsprung, gekoppelt werden, und anschließend an dem Finger, insbesondere dem Daumen, des Patienten beispielsweise durch Umwickeln desselben angebracht werden. Der Vorsprung kann zumindest in einem Querschnitt in dorsaler Richtung T-förmig oder pilzförmig ausgebildet sein. Der Koppelabschnitt kann ferner Klettverschluss zum Koppeln mit dem zweiten Orthesenabschnitt umfassen.

Der Anlageabschnitt kann zum seitlichen Anliegen an dem Finger vorgesehen sein. Dadurch kann eine verbesserte Stabilisierung des Fingers bewirkt werden.

Der Anlageabschnitt kann relativ zu einem proximalen Abschnitt des Schienenelements um eine Längsachse des Schienenelements tordiert angeordnet sein. Dies ermöglicht es, dass der Anlageabschnitt beispielsweise zum seitlichen Anliegen an dem Finger vorgesehen ist, während der proximale Abschnitt zur dorsalen Anordnung, insbesondere zum dorsalen Anliegen, an der Hand, insbesondere Handrücken oder Fingerrücken, vorgesehen ist. Diese Anordnung ermöglicht eine seitliche Krafteinleitung an dem Finger, wobei gleichzeitig die handrückenseitige Anordnung des proximalen Abschnitts eine weitgehend freie Bewegbarkeit der Hand erlaubt.

Gemäß einer Weiterbildung ist eine räumliche Erstreckung des ersten Orthesenabschnitts in einem an der Hand des Patienten angebrachten Zustand auf den Mittelhandbereich beschränkt. Dies bedeutet beispielsweise, dass der erste Orthesenabschnitt nicht an einem oder mehreren Fingern, dem Handgelenk der Hand oder einem Unterarmbereich angebracht ist. Vereinfacht gesagt ist vorgesehen, dass der erste Orthesenabschnitt nicht an anderen Körperteilen des Patienten außer dem Mittelhandbereich der Hand angebracht wird.

Gemäß einer Ausgestaltung der Erfindung kann vorgesehen sein, dass der erste Orthesenabschnitt eine derartige räumliche Erstreckung aufweist, dass ein Handgelenk der Hand frei beweglich ist und/oder das Handgelenk durch den ersten Orthesenabschnitt nicht überdeckt ist. Es versteht sich, dass damit Eigenschaften der Handorthese beschrieben sind, wenn die Handorthese an der Hand des Patienten angebracht ist.

Gemäß einer Weiterbildung kann vorgesehen sein, dass der erste Orthesenabschnitt eine derartige räumliche Erstreckung aufweist, dass ein Daumengrundgelenk und/oder ein Daumenendgelenk der Hand des Patienten von dem ersten Orthesenabschnitt nicht überdeckt ist.

Gemäß einer Weiterbildung der Erfindung kann der erste Orthesenabschnitt öffenbar und/oder an eine Größe der Hand des Patienten anpassbar ausgebildet sein. Dazu kann der erste Orthesenabschnitt einen bevorzugt öffenbaren Verschließmechanismus umfassen. Ein solcher Verschließmechanismus kann einen Klettverschluss, Reißverschluss, Magnetverschluss und/oder zumindest ein Band umfassen.

Gemäß einer Ausgestaltung der Erfindung ist der erste Orthesenabschnitt im Wesentlichen einstückig ausgebildet. Ferner kann der erste Orthesenabschnitt elastisch ausgebildet sein.

Gemäß einem weiteren Aspekt der Erfindung umfasst der erste Orthesenabschnitt ferner eine zweite Öffnung zur Aufnahme zumindest eines weiteren oder bevorzugt der übrigen Finger der Hand. Ist die Handorthese als eine Daumenorthese ausgebildet, so ist die erste Öffnung zum Einführen bzw. Aufnehmen des Daumens und die zweite Öffnung beispielsweise zum Einführen bzw. Aufnehmen eines weiteren Fingers, bevorzugt der übrigen Finger, der Hand des Patienten vorgesehen.

Gemäß einem Aspekt der Erfindung umfasst der erste Orthesenabschnitt ferner eine dritte Öffnung zur Aufnahme eines Handwurzelbereichs und/oder eines Handgelenkbereichs. Dabei kann die dritte Öffnung derart groß ausgebildet sein, dass der Patient zum Anlegen der Handorthese die Hand durch die dritte Öffnung und anschließend den Finger durch die erste Öffnung einführen kann. Ferner kann der erste Orthesenabschnitt zumindest im Bereich der dritten Öffnung elastisch ausgebildet sein. Dadurch ist die dritte Öffnung zum Durchführen der Hand elastisch dehnbar.

Es versteht sich, dass die Öffnungen nicht abgeschlossen an der Handorthese ausgebildet sein müssen. So kann die Handorthese beispielsweise zwischen zumindest zwei solcher Öffnungen öffenbar, insbesondere mit einem Verschließmechnanismus zum Öffnen und Schließen der Handorthese, ausgebildet sein. Dies erleichtert das Anlegen der Handorthese an der Hand. Eine jeweilige Öffnung beschreibt lediglich eine Öffnung, die vorzugsweise im an der Hand angelegten Zustand der Handorthese ausgebildet ist. Ferner kann vorgesehen sein, dass benachbarte Öffnungen ineinander übergehen.

Gemäß einer Weiterbildung der Erfindung ist der zweite Orthesenabschnitt dazu eingerichtet, positionstreu relativ zu dem Mittelhandbereich angebracht zu werden.

Gemäß einer Ausgestaltung der Erfindung kann vorgesehen sein, dass der zweite Orthesenabschnitt dazu eingerichtet ist, positionstreu relativ zu dem Finger angebracht zu werden.

Gemäß einer Weiterbildung der Erfindung kann der zweite Orthesenabschnitt dazu vorgesehen sein, im Wesentlichen an und/oder proximal zu einem Fingermittelgelenk oder Fingerendgelenk angebracht zu werden. Betrifft die Handorthese eine Daumenorthese, so kann vorgesehen sein, dass der zweite Orthesenabschnitt im Bereich des Daumenendgelenks, bevorzugt überwiegend proximal des Daumenendgelenks und/oder distal des Daumengrundgelenks, an dem Daumen anbringbar ist. Bevorzugt ist der zweite Orthesenabschnitt zur Anbringung an und/oder proximal des Daumenendgelenks vorgesehen. Üblicherweise ist der Bereich des Daumenendgelenks an dem Daumen dicker ausgebildet als der Bereich proximal und distal des Daumenendgelenks. Ist der zweite Orthesenabschnitt proximal zu dem Daumenendgelenk angebracht, so kann die Daumenorthese eine Kraft proximal des Daumenendgelenks auf den Daumen aufbringen und den Daumen in Richtung distal relativ zu dem ersten Orthesenabschnitt drücken. Dadurch kann die Traktion auf das Daumensattelgelenk und/oder das Daumengrundgelenk aufgebracht werden, um die schmerzlindernde Wirkung zu erzielen.

Gemäß einer Weiterbildung der Erfindung kann vorgesehen sein, dass der zweite Orthesenabschnitt in dem am Finger angebrachten Zustand nur einen Teil des Fingers in Fingerlängsrichtung umgreift. Ist die Handorthese als Daumenorthese ausgebildet, kann der zweite Orthesenabschnitt dazu vorgesehen sein, lediglich einen Bereich zwischen dem Daumenendgelenk und dem Daumengrundgelenk zu umgreifen.

Gemäß einer Weiterbildung der Erfindung kann der zweite Orthesenabschnitt öffenbar und/oder an eine Größe des Fingers anpassbar ausgebildet sein. Dazu kann der zweite Orthesenabschnitt einen bevorzugt öffenbaren Verschließmechanismus umfassen. Ein solcher Verschließmechanismus kann einen Klettverschluss, Reißverschluss, Magnetverschluss und/oder zumindest ein Band umfassen.

Gemäß einer Weiterbildung der Erfindung kann vorgesehen sein, dass der zweite Orthesenabschnitt im Wesentlichen einstückig ausgebildet ist.

Gemäß einer Weiterbildung der Erfindung kann vorgesehen sein, dass der zweite Orthesenabschnitt elastisch ausgebildet ist. Dies kann es erleichtern, den zweiten Orthesenabschnitt an dem Finger anzubringen. So kann der zweite Orthesenabschnitt beim Anlegen der Orthese beispielsweise über den Finger an die beabsichtige Position geschoben werden. Ferner kann dadurch der Tragekomfort erhöht werden.

Gemäß einer alternativen Ausgestaltung der Erfindung ist der zweite Orthesenabschnitt unelastisch ausgebildet. So kann der zweite Orthesenabschnitt gezielt an eine Größe eines Fingers angepasst werden.

Gemäß einer Ausgestaltung der Erfindung kann vorgesehen sein, dass die Handorthese ferner eine zweite Schienenanordnung umfasst. Es können auch weitere Schienenanordnungen vorgesehen sein. Die vorangehend beschriebene Schienenanordnung ist damit die erste Schienenanordnung. Diese zweite Schienenanordnung oder die weiteren Schienenanordnungen können wie die erste Schienenanordnung ausgebildet sein. Ferner kann die zweite Schienenanordnung oder die weiteren Schienenanordnungen wie vorangehend zur ersten Schienenanordnung beschrieben ausgebildet sein. So kann die zweite Schienenanordnung den ersten Orthesenabschnitt und den zweiten Orthesenabschnitt aneinanderkoppeln und ferner dazu vorgesehen sein, den zweiten Orthesenabschnitt in einer vorgebbaren Lage relativ zu dem ersten Orthesenabschnitt anzuordnen. Im Rahmen dieser Ausgestaltung kann vorgesehen sein, dass die zweite Schienenanordnung ein längliches Schienenelement und/oder eine zugehörige betätigbare Verstellanordnung umfasst, die jeweils wie vorangehend zur ersten Schienenanordnung beschrieben ausgebildet und eingerichtet sind.

Gemäß einer Weiterbildung der Erfindung kann vorgesehen sein, dass die erste Schienenanordnung an einem dorsalseitigen Bereich des ersten und/oder zweiten Orthesenabschnitts und die zweite Schienenanordnung an einem palmarseitigen Bereich des ersten und/oder zweiten Orthesenabschnitts angeordnet ist.

Gemäß einer Ausgestaltung der Erfindung kann vorgesehen sein, dass zumindest die erste und die zweite Schienenanordnung im Wesentlichen parallel zueinander und/oder parallel relativ zu einer Fingerlängsrichtung ausgerichtet sind.

Gemäß einer Weiterbildung der Erfindung kann vorgesehen sein, dass der zweite Orthesenabschnitt von dem ersten Orthesenabschnitt entkoppelbar ausgebildet ist. Dazu kann die Schienenanordnung von dem ersten und/oder zweiten Orthesenabschnitt trennbar ausgebildet sein. Zusätzlich oder alternativ kann die Schienenanordnung trennbar ausgebildet sein. Beispielsweise kann vorgesehen sein, dass das erste längliche Schienenelement von dem ersten und/oder dem zweiten Orthesenabschnitt und/oder dem zweiten länglichen Schienenelement trennbar ausgebildet ist. Dies erlaubt es, dass die Handorthese zumindest zeitweise mit weniger Komponenten ausgebildet ist und beispielsweise der zweite Orthesenabschnitt entfernt wird. Dann kann die Handorthese zumindest zeitweise zumindest den ersten Orthesenabschnitt umfassen und mit diesem lediglich an dem Mittelhandbereich der Hand des Patienten angebracht sein. Anders ausgedrückt erlaubt dies, die Handorthese zumindest zeitweilig auf eine Handorthese zur Therapie der Mittelhand zu reduzieren und bedarfsabhängig zur Traktion des Fingergelenks zu erweitern.

Die Erfindung wird nachfolgend anhand der beigefügten Zeichnungen weiter erläutert. Es stellen dar:
- Fig. 1: eine schematische Ansicht einer Handorthese gemäß einem ersten Ausführungsbeispiel;
- Fig. 2: eine schematische Ansicht einer Handorthese gemäß einem zweiten Ausführungsbeispiel;
- Fig. 3a: eine Detaildarstellung einer Verstellanordnung gemäß einem Ausführungsbeispiel;
- Fig. 3b: eine weitere Detaildarstellung der Verstellanordnung gemäß dem Ausführungsbeispiel;
- Fig. 4: eine schematische Ansicht einer Handorthese gemäß einem dritten Ausführungsbeispiel;
- Fig. 5: eine schematische Ansicht einer Schienenanordnung gemäß einem weiteren Ausführungsbeispiel; und
- Fig. 6: eine schematische Ansicht einer Schienenanordnung gemäß einem weiteren Ausführungsbeispiel.

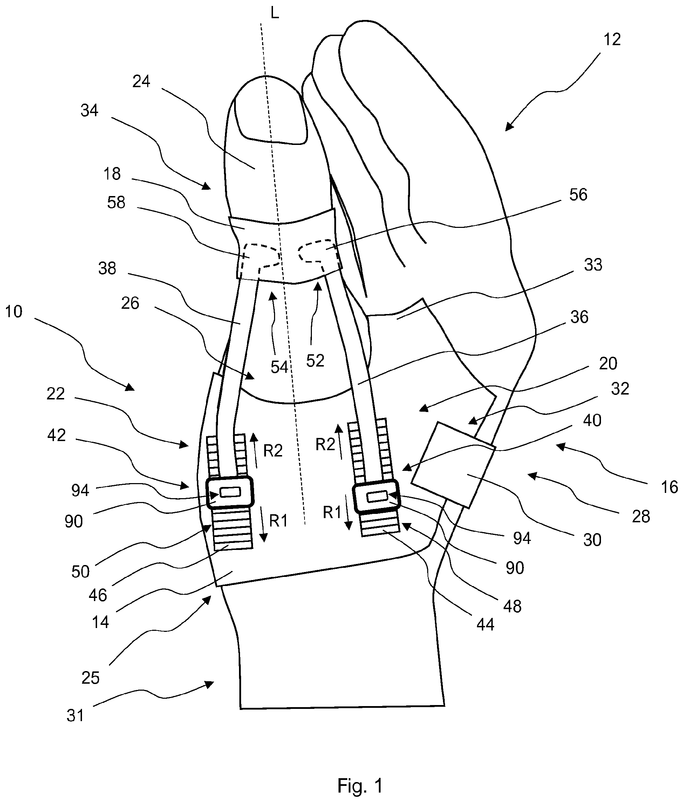

Figur 1 zeigt eine Handorthese 10 zur Traktion eines Fingergelenks gemäß einem ersten Ausführungsbeispiel. Die Handorthese 10 ist an einer Hand 12 eines Patienten angelegt. Die Handorthese 10 umfasst einen ersten Orthesenabschnitt 14 zum Anbringen an einem Mittelhandbereich 16 der Hand 12 des Patienten, einen zweiten Orthesenabschnitt 18 zum Anbringen an einem Finger der Hand 12 des Patienten und eine erste Schienenanordnung 20 und eine zweite Schienenanordnung 22.

Vorliegend ist die Handorthese 10 als Daumenorthese ausgebildet. Dementsprechend ist der zweite Orthesenabschnitt 18 an einem Daumen 24 der Hand 12 des Patienten angebracht. Je nachdem, welcher Finger therapiert werden soll, könnte die Handorthese 10 auch anders ausgebildet sein, sodass der zweite Orthesenabschnitt 18 zum Anbringen an dem entsprechenden anderen Finger der Hand 12 vorgesehen ist.

Der erste Orthesenabschnitt 14 umfasst eine erste Öffnung 26 zum Einführen bzw. Aufnehmen des Daumens 24 der Hand 16 des Patienten. Diese erste Öffnung 26 ist dazu vorgesehen, den Daumen 24 bevorzugt an dessen proximalen Ansatz und vollständig zu umgeben. Ferner umfasst der erste Orthesenabschnitt 14 zumindest einen Verschließmechanismus 28. Beispielhaft weist der Verschließmechanismus 28 einen Gurt 30 auf, der einerseits an dem ersten Orthesenabschnitt 14 auf einer nicht dargestellten Seite der Handorthese 10 fest angebracht ist und andererseits an dem ersten Orthesenabschnitt 14 anbringbar ausgebildet ist. Vorliegend weist der Gurt 30 eine Klettverschlussanordnung 32 auf, die mit dem ersten Orthesenabschnitt 14 verbindbar ist. Zusätzlich oder statt eines solchen Verschließmechanismus 28 könnte der erste Orthesenabschnitt 14 auch großflächiger ausgebildet und/oder zum selbstbefestigenden Anliegen an der Hand 12 vorgesehen sein, beispielsweise in der Art eines Handschuhs, der zumindest einen Teil des Mittelhandbereichs 16 umgibt und durch Aufziehen bzw. Anlegen an diesen positionstreu angebracht werden kann.

Der erste Orthesenabschnitt 14 bzw. eine räumliche Erstreckung desselben ist vorzugsweise wie vorliegend auf den Mittelhandbereich 16 beschränkt. Dies bedeutet, dass der erste Orthesenabschnitt 14 beispielsweise einen Handgelenkbereich 31, den Daumen 24 und/oder andere Finger der Hand 12 nicht überlagert. Der erste Orthesenabschnitt 14 umfasst auch einen nicht dargestellten, handinnenseitigen Bereich, an dem vorliegend der Gurt 30 angebracht ist. Der Gurt 30 erstreckt sich somit über einen Handrücken der Hand 12 bis zu dem handinnenseitigen Bereich des ersten Orthesenabschnitts 14. Der erste Orthesenabschnitt 12 umfasst einen Übergangsabschnitt 33, der an der ersten Öffnung 26 ausgebildet ist und dazu vorgesehen ist, in dem an der Hand 12 angeordneten Zustand der Handorthese 10 zwischen dem Daumen 24 und den übrigen Fingern der Hand 12 angeordnet zu sein. Der Übergangsabschnitt 33 verbindet den nicht dargestellten handinnenseitigen Bereich des ersten Orthesenabschnitts 14 mit dem dargestellten, handrückenseitigen Bereich des ersten Orthesenabschnitts 14.

Der zweite Orthesenabschnitt 18 ist getrennt von und beabstandet zu dem ersten Orthesenabschnitt 14 ausgebildet. Der zweite Orthesenabschnitt 18 ist ferner ringförmig ausgebildet und umgibt den Daumen 24 in Umfangsrichtung vollständig und proximal eines Daumenendgelenks 34 der Hand 12. Man könnte auch sagen, dass der zweite Orthesenabschnitt 18 zumindest zum Anliegen proximal an den Kondylen des Daumenendgelenks 34 und/oder infrakondylär vorgesehen ist. Der zweite Orthesenabschnitt 18 ist zumindest bis zu einem gewissen Grad elastisch ausgebildet, sodass der zweite Orthesenabschnitt 18 zum Anlegen der Handorthese 10 über den Daumen 24 gezogen werden kann, er sich dabei weitet und anschließend an die Daumenform angepasst proximal des Daumenendgelenks 34 an dem Daumenendgelenk 34 anliegt. Der zweite Orthesenabschnitt 18 könnte öffenbar und verschließbar ausgebildet sein, beispielsweise mit einem Klettverschluss, um den zweiten Orthesenabschnitt 18 einfach an dem Daumen 24 anzubringen und an die tatsächliche Größe des Daumens 24 anzupassen.

Der erste Orthesenabschnitt 14 und der zweite Orthesenabschnitt 18 umfassen jeweils ein flächiges Stoffmaterial, beispielsweise umfassend Filz, Baumwolle, Kunstfaser etc. Dies ermöglicht einen angenehmen Tragekomfort für den Patienten.

Die erste Schienenanordnung 20 und die zweite Schienenanordnung 22 sind vorliegend im Wesentlichen gleichartig ausgebildet. So umfassen die Schienenanordnungen 20, 22 jeweils ein erstes längliches Schienenelement 36, 38, das jeweils mit dem zweiten Orthesenabschnitt 18 gekoppelt ist. Ferner umfassen die Schienenanordnungen 20, 22 jeweils eine betätigbare Verstellanordnung 40, 42, die jeweils ein Verstellteil 90 und eine Betätigungsanordnung 94 umfassen. Die Funktionsweise der Verstellanordnung 40, 42, des jeweiligen Verstellteils 90 und der jeweiligen Betätigungsanordnung 94 wird noch im Folgenden näher beschrieben werden.

Die jeweilige Verstellanordnung 40, 42 ist dazu eingerichtet, bei Betätigung ein Ändern der Lage des jeweiligen ersten länglichen Schienenelements 36, 38 und dem damit gekoppelten zweiten Orthesenabschnitt 18 relativ zu dem ersten Orthesenabschnitt 14 zuzulassen. Dazu umfasst die jeweilige Schienenanordnung 20, 22 in der vorliegenden Ausführungsform ein zweites längliches Schienenelement 44, 46, das mit dem ersten Orthesenabschnitt 14 gekoppelt ist. Man könnte auch sagen, dass das zweite längliche Schienenelement 44, 46 vorliegend an dem ersten Orthesenabschnitt 14 fest angebracht ist. Das jeweilige zweite längliche Schienenelement 44, 46 umfasst eine Mehrzahl an Rastnuten 48, 50, in die die Verstellanordnung 40, 42 eingreifen kann, um die Lage der beiden Orthesenabschnitte 14, 18 relativ zueinander vorzugeben. Die Verstellanordnung 40, 42 ist mit dem jeweiligen ersten länglichen Schienenelement 36, 38 gekoppelt und relativ zu und an dem jeweiligen zweiten länglichen Schienenelement 44, 46 verschieblich gelagert. Ist die Verstellanordnung 40, 42 nicht betätigt, so rastet die Verstellanordnung 40, 42 in einer jeweiligen Rastnut 48, 50 ein, sodass die Lage der zweiten Orthesenabschnitts 18 relativ zu dem ersten Orthesenabschntit 14 vorgegeben ist. Ein Ändern der Lage der Verstellanordnung 40, 42 ist dann nicht oder zumindest nur in einer zweiten Richtung R2 zugelassen, wohingegen ein Ändern der Lage der Verstellanordnung 40, 42 in einer ersten Richtung R1 relativ zu dem jeweiligen zweiten länglichen Schienenelement 44, 46 nicht zugelassen ist. Ist die Verstellanordnung 40, 42 betätigt, so rastet die Verstellanordnung 40, 42 nicht in eine der Rastnuten 48, 50. Somit ist ein Ändern der Lage der Verstellanordnung 40, 42 relativ zu dem jeweiligen zweiten länglichen Schienenelement 44, 46 zugelassen, gemäß der vorliegenden Ausführungsform in der ersten Richtung R1 und in der zweiten Richtung R2. Die Lage des zweiten Orthesenabschnitts 18 relativ zu dem ersten Orthesenabschnitt 14 kann vorgegeben werden, indem die Lage verändert wird und die jeweilige Verstellanordnung 40, 42 in eine gewünschte der Rastnuten 48, 50 eingreift. Die Vorgabe der Lage beeinflusst die Traktion auf das Fingergelenk. Dieses Prinzip ist ähnelt einem betätigbaren Kabelbinderverschluss. Die zweite Richtung R2 ist entgegengesetzt zu der ersten Richtung R1 ausgerichtet. Die zweite Richtung R2 ist im Wesentlichen gemäß einer Daumenlängsachse L, die auch als Fingerlängsrichtung bezeichnet sein kann, ausgerichtet und in Richtung des Daumens 24 orientiert.

Das jeweilige erste längliche Schienenelement 36, 38 ist länglich ausgebildet und an die Form des Daumens 24 angepasst. In der vorliegenden Ausführungsform ist die erste Schienenanordnung 20 einerseits der Daumenlängsachse L ausgebildet und die zweite Schienenanordnung 22 andererseits der Daumenlängsachse L ausgebildet. Man könnte auch sagen, dass die erste Schienenanordnung 20 lateral des Daumens 24 und die zweite Schienenanordnung 22 medial des Daumens 24 angeordnet ist. Die beiden zweiten länglichen Schienenelemente 44, 46 sind im Wesentlichen parallel zueinander an dem ersten Orthesenabschnitt 14 ausgerichtet, sodass die jeweilige erste Richtung R1 und/oder zweite Richtung R2 gleich ausgerichtet ist.

Das jeweilige erste längliche Schienenelement 36, 38 weist eine erhöhte Steifigkeit auf und/oder umfasst einen Kunststoffwerkstoff, einen metallischen Werkstoff, Holz und/oder einen Faserverbundwerkstoff. Insgesamt ist die erste und/oder die zweite Schienenanordnung 20, 22 steifer ausgebildet als der erste und der zweite Orthesenabschnitt 14, 18. Die erste und/oder die zweite Schienenanordnung 20, 22 sind vorzugsweise biegesteif und/oder torsionssteif ausgebildet. Die erste und/oder die zweite Schienenanordnung 20, 22 weist vorzugsweise einen Kunststoffwerkstoff, einen metallischen Werkstoff, Holz und/oder einen Faserverbundwerkstoff auf. Die Ausführung mit den beiden Schienenanordnungen 20, 22 führt zu einer höheren Biegesteifigkeit und Torsionssteifigkeit als eine Ausführung der Handorthese 10 mit lediglich einer Schienenanordnung.

Der zweite Orthesenabschnitt 18 umfasst eine erste Tasche 52 zur Aufnahme eines ersten Endes 56 des ersten länglichen Schienenelements 36 der ersten Schienenanordnung 20 und eine zweite Tasche 54 zur Aufnahme eines ersten Endes 58 der zweiten Schienenanordnung 22. Die Enden 56, 58 sind jeweils flächig und zum Anliegen an dem Finger bzw. Daumen 24 ausgebildet, um ein flächiges Anliegen an dem Daumen 24 im Bereich des zweiten Orthesenabschnitts 18 bereitzustellen. Vorliegenden sind die Enden 56, 58 P-förmig ausgebildet, könnten aber auch T-, D-, O-förmig oder ähnlich oder halbkreisförmig den Daumen 24 in Umfangsrichtung im Wesentlichen hälftig umfassend ausgebildet sein. Statt der zwei Taschen 52, 54 könnte auch eine gemeinsame Tasche an dem zweiten Orthesenabschnitt 18 ausgebildet sein. Ferner könnten statt der zwei Taschen 52, 54 die jeweiligen Enden 56, 58 auch lediglich daumenseitig zu dem zweiten Orthesenabschnitt 18 angeordnet sein und von dem zweiten Orthesenabschnitt 18 an den Finger geklemmt werden. Alternativ könnten die Enden 56, 58 auch auf eine andere Art mit dem zweiten Orthesenabschnitt 218 gekoppelt sein, beispielsweise geklebt, verschweißt, genietet etc. Ein Vorteil der Ausgestaltung mit zwei Schienenanordnungen und entsprechenden zwei Enden 56, 58 ist, dass der zweite Orthesenabschnitt 14 gegenüber einer Lösung, bei der ein Ende den Daumen 24 in Umfangsrichtung vollständig umgreift, besser an die Größe des Daumens 24, insbesondere die Größe der Kondylen am Daumenendgelenk 34, anpassbar ist.

Um das Ausmaß der Traktion auf das beabsichtigte Fingergelenk der Hand 12 anzupassen, kann die Lage der jeweilige Verstellanordnung 40, 42 relativ zu dem jeweiligen zweiten länglichen Schienenelement 44, 46 bzw. dem ersten Orthesenabschnitt 14 geändert werden. Dadurch wird auch die Lage des jeweiligen ersten länglichen Schienenelements 36, 38 und damit des zweiten Orthesenabschnitts 18 relativ zu dem ersten Orthesenabschnitt 14 geändert. Wird nun die Lage derart geändert, dass die beiden Orthesenabschnitte 14, 18 auseinander bewegt werden, so wird auf den Finger, vorliegen auf den Daumen 24, mittels des zweiten Orthesenabschnitts 18 eine Kraft ausgeübt, die in distaler Richtung wirkt, wohingegen auf den Mittelhandbereich 16 mittels des ersten Orthesenabschnitts 14 eine Kraft ausgeübt wird, die in medialer Richtung wirkt. Man könnte auch sagen, dass der Finger, vorliegend der Daumen 24, in Richtung distal und der Mittelhandbereich 16 in Richtung medial gedrückt werden. Dadurch wirkt auf das Fingergelenk, vorliegend auf das nicht näher dargestellte Daumensattelgelenk 25, eine Zugkraft bzw. die Traktion. Mittels der Verstellanordnung 40, 42 kann also das Ausmaß der Traktion eingestellt werden.

Ein Vorteil der Lösung mit den beiden Schienenanordnungen 20, 22 liegt darin, dass diese unabhängig voneinander eingestellt werden können und diese ferner in Umfangrichtung des Daumens 24 an unterschiedlichen Positionen im Bereich des zweiten Orthesenabschnitts 18 angreifen. Damit kann die Position, in die der Daumen 24 sozusagen mittels der Schienenanordnungen 20, 22 gedrückt wird, angepasst werden. Je länger die erste Schienenanordnung 20 und/oder je kürzer die zweite Schienenanordnung 22 eingestellt wird, desto mehr wird der Daumen 24 gewünschten Falls zusätzlich zu einer rein distalen Traktionsbelastung in Richtung weg von den übrigen Fingern der Hand 12 gedrückt und umgekehrt. Dadurch kann eine zusätzliche Traktion auf das Daumensattelgelenk bewirkt werden.

Es versteht sich, dass auch nur eine der Schienenanordnungen 20, 22 oder auch drei, vier oder mehr solcher Schienenanordnungen vorgesehen sein könnten. Bevorzugt könnte zumindest eine palmarseitige Schienenanordnung vorgesehen sein.

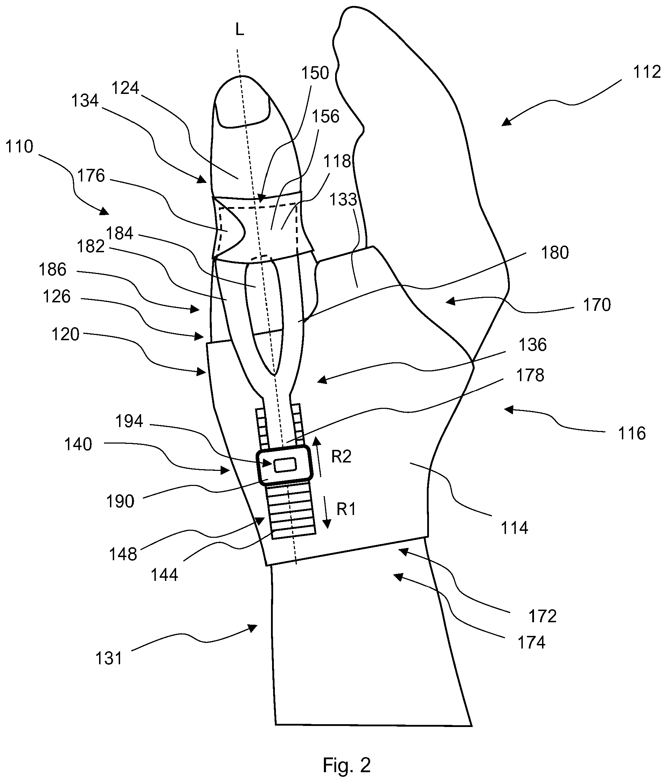

Figur 2 zeigt eine schematische Ansicht einer Handorthese 110 gemäß einem zweiten Ausführungsbeispiel. Wie auch die Handorthese 10 gemäß Figur 1, umfasst die Handorthese 110 einen ersten Orthesenabschnitt 114 zum Anbringen an einem Mittelhandbereich 116 einer Hand 112 des Patienten, einen zweiten Orthesenabschnitt 118 zum Anbringen an einem Finger der Hand 112 des Patienten und eine erste Schienenanordnung 120.

Die Handorthese 110 ist ähnlich wie die Handorthese 10 gemäß Figur 1 aufgebaut, wobei im Folgenden besonders auf Unterschiede eingegangen werden soll. Gleiche oder ähnliche Merkmale sind mit einem gleichen Bezugszeichen addiert mit 100 dargestellt.

Vorliegend ist die Handorthese 110 beispielhaft als Daumenorthese ausgebildet. Dementsprechend ist der zweite Orthesenabschnitt 118 an einem Daumen 124 der Hand 112 des Patienten angebracht.

Der erste Orthesenabschnitt 114 umfasst eine erste Öffnung 126 zum Einführen bzw. Aufnehmen des Daumens 224 der Hand 116 des Patienten. Diese erste Öffnung 126 ist dazu vorgesehen, den Daumen 224 bevorzugt an dessen proximalen Ansatz und vollständig zu umgeben. Wird die Handorthese 110 an der Hand 112 des Patienten angelegt, dann wird der Daumen 124 durch die erste Öffnung 126 geführt. Der erste Orthesenabschnitt 114 umfasst ferner zumindest eine zweite Öffnung 170 zur Aufnahme der übrigen Finger der Hand 112, also der Finger außer dem Daumen 124. Die zweite Öffnung 170 ist dazu vorgesehen, einen proximalen Ansatz der übrigen Finger bevorzugt vollständig zu umgeben. Wird die Handorthese 110 an der Hand 112 des Patienten angelegt, dann werden die übrigen Finger der Hand 112 durch die zweite Öffnung 170 geführt. Der erste Orthesenabschnitt 114 umfasst ferner eine dritte Öffnung 172 zur Aufnahme eines Handwurzelbereichs 174 der Hand des Patienten. Die dritte Öffnung 172 ist dazu vorgesehen, den Handwurzelbereich 174 in Umfangsrichtung der Hand 112 bevorzugt vollständig zu umgeben. Wird die Handorthese 110 an der Hand 112 des Patienten angelegt, dann werden zunächst alle Finger der Hand 112 durch die dritte Öffnung 172 geführt. Anschließend werden der Daumen 124 durch die erste Öffnung 126 und die übrigen Finger durch die zweite Öffnung 170 geführt, bis der erste Orthesenabschnitt 114 an dem Mittelhandbereich 116 anliegt und die dritte Öffnung 172 den Handwurzelbereich 174 umgibt. Bevorzugt ist der erste Orthesenabschnitt 114 zumindest im Bereich der dritten Öffnung 172 elastisch ausgebildet, sodass er sich an die Form des Mittelhandbereichs 116 anpassen kann. Zusätzlich oder alternativ kann die Form des ersten Orthesenabschnitts 114 an die Form des Mittelhandbereichs 116 angepasst sein. Der erste Orthesenabschnitt 114 umgibt den Mittelhandbereich 116 in Umfangsrichtung vollständig. Der erste Orthesenabschnitt 114 bzw. eine räumliche Erstreckung des ersten Orthesenabschnitts 114 ist auf den Mittelhandbereich 116 beschränkt. Dies bedeutet, dass der erste Orthesenabschnitt 14 beispielsweise einen Handgelenkbereich 131, den Daumen 124 und/oder andere Finger der Hand 112 nicht überlagert.

Ähnlich wie bei der ersten Ausführungsform der Handorthese kann der erste Orthesenabschnitt 114 öffenbar ausgebildet sein und beispielsweise einen Verschließmechanismus umfassen. Ein solches Verschließmechanismus könnte an dem ersten Orthesenabschnitt 114 im Bereich der dritten Öffnung 126 vorgesehen sein, um diesen Bereich an den Umfang der Hand 112 anzupassen, die Handorthese 112 zum Anlegen zu öffnen und anschließend zu schließen.

Der erste Orthesenabschnitt 112 umfasst einen Übergangsabschnitt 133, der zwischen der ersten Öffnung 126 und der zweiten Öffnung 170 ausgebildet ist und dazu vorgesehen ist, in dem an der Hand 112 angeordneten Zustand der Handorthese 110 zwischen dem Daumen 124 und den übrigen Fingern der Hand 112 angeordnet zu sein. Der Übergangsabschnitt 133 verbindet den nicht dargestellten handinnenseitigen Bereich des ersten Orthesenabschnitts 114 mit dem zumindest teilweise dargestellten, handrückenseitigen Bereich des ersten Orthesenabschnitts 114. Der Übergangsabschnitt 113 ist in besonderem Maße dazu vorgesehen, eine proximal wirkende Kraft, die von der ersten Schienenanordnung 120 auf den ersten Orthesenabschnitt 114 aufgebracht werden kann, auf die Hand 112, insbesondere den Mittelhandbereich 116, zu übertragen.

Der zweite Orthesenabschnitt 118 ist ringförmig ausgebildet und umgibt den Daumen 124 in Umfangsrichtung vollständig und proximal des Daumenendgelenks 134. Man könnte auch sagen, dass der zweite Orthesenabschnitt 118 zumindest zum Anliegen an den proximalen Kondylen des Daumenendgelenks 134 vorgesehen ist. Der zweite Orthesenabschnitt 118 ist öffenbar und verschließbar ausgebildet. Dies ermöglicht es, den zweiten Orthesenabschnitt 118 einfach an dem Daumen 124 anzubringen und an die tatsächliche Größe des Daumens 124 anzupassen. Zum Öffnen und Schließen umfasst der zweite Orthesenabschnitt 118 ein Ende, das eine Lasche 176 umfasst, wobei der zweite Orthesenabschnitt 118 beim Anlegen desselben in Umfangsrichtung um den Daumen 124 gelegt und die Lasche 176 mit einer Außenseite des zweiten Orthesenabschnitts 118 je nach Daumengröße oder Patientenbelieben gekoppelt werden kann. Die Lasche 176 umfasst dazu eine Art Klettverschluss, der mit der Außenseite des zweiten Orthesenabschnitts 118 koppelbar ist. Der zweite Orthesenabschnitt 118 ist getrennt von dem ersten Orthesenabschnitt 114 ausgebildet und beabstandet zu dem ersten Orthesenabschnitt 114 angeordnet.

Das zweite längliche Schienenelement 144 der ersten Schienenanordnung 120 ist wie vorangehend zur Figur 1 beschrieben ausgebildet und umfasst eine Mehrzahl an Rastnuten 148. Ferner weist das erste längliche Schienenelement 136 in dem Bereich, der dem zweiten länglichen Schienenelement 144 zuordenbar ist, einen proximalen Abschnitt 178 auf, der auch als Grundabschnitt bezeichnet werden kann. Der proximale Abschnitt 178 weist eine im Wesentlichen konstante Breite auf. Distal dieses proximalen Abschnitts 178 sind zwei im Wesentlichen parallel zueinander ausgerichtete Gabelabschnitte 180, 182 an dem ersten länglichen Schienenelement 136 ausgebildet, wobei das erste längliche Schienenelement 136 ferner ein distales Ende 156 aufweist. Zwischen den Gabelabschnitten 180, 182 ist eine Aussparung 184 ausgebildet, die wie vorliegend an einem Daumenrücken angeordnet und im Bereich eines Fingergelenks, vorliegen im Bereich eines Daumengrundgelenks 186, ausgebildet sein kann. Die Aussparung 184 dient der Gewichtsersparnis. Ferner tragen die beiden Gabelabschnitte 180, 182 zu einer erhöhten Stabilität bei. Daneben kann die Aussparung 184 ein Beugen des Daumens 124 erleichtern, da das erste längliche Schienenelement 136 so nicht oder kaum mit dem Daumengrundgelenk 186 des Daumens 124 beim Beugen des Daumens 124 kollidiert. Stattdessen können die Gabelabschnitte 180, 182 bei Betrachtung in Richtung auf den Handrücken der Hand 112 bei Beugung des Daumens 124 medial und lateral des Daumengrundgelenks 186 verlaufen, ohne mit dem Daumengrundgelenk 186 zu kollidieren. Die erste Öffnung 126 ist vorzugsweise derart ausgebildet, dass der Bereich des Daumengrundgelenks 186 im Wesentlichen freiliegt und von dem ersten Orthesenabschnitt 114 nicht überlagert ist.

Der zweite Orthesenabschnitt 118 weist eine Tasche 150 zur Aufnahme des distalen Endes 156 des ersten länglichen Schienenelements 136 auf. So ist die Tasche 150 in proximaler Richtung des zweiten Orthesenabschnitts 118 zur Aufnahme des distalen Endes 156 des ersten länglichen Schienenelements 136 ausgebildet, wohingegen die Tasche 150 in distaler Richtung geschlossen ausgebildet ist, damit das distale Ende 156 eine distale Kraft auf den zweiten Orthesenabschnitt 118 ausüben kann.

Die erste Schienenanordnung 120 umfasst eine betätigbare Verstellanordnung 140. Die betätigbare Verstellanordnung 140 ist wie vorangehend zur Figur 1 beschrieben ausgebildet und mit dem ersten länglichen Schienenelement 136 gekoppelt und relativ zu und an dem zweiten länglichen Schienenelement 144 verschieblich gelagert. Ferner umfasst die Verstellanordnung 140 ein Verstellteil 190 und eine Betätigungsanordnung 194 zum Betätigen der Verstellanordnung 140. Die Funktionsweise der Verstellanordnung 140, des Verstellteils 190 und der Betätigungsanordnung 194 wird noch im Folgenden näher beschrieben werden.

Anders als der Handorthese 10 der ersten Ausführungsform weist die Handorthese 110 lediglich eine Schienenanordnung 120 auf, wobei optional auch eine zweite Schienenanordnung, optional auch weitere solche Schienenanordnungen, vorgesehen sein könnten. Eine zweite Schienenanordnung könnte beispielsweise palmar oder medial relativ zu der ersten Schienenanordnung 120 an der Handorthese 110 vorgesehen sein.

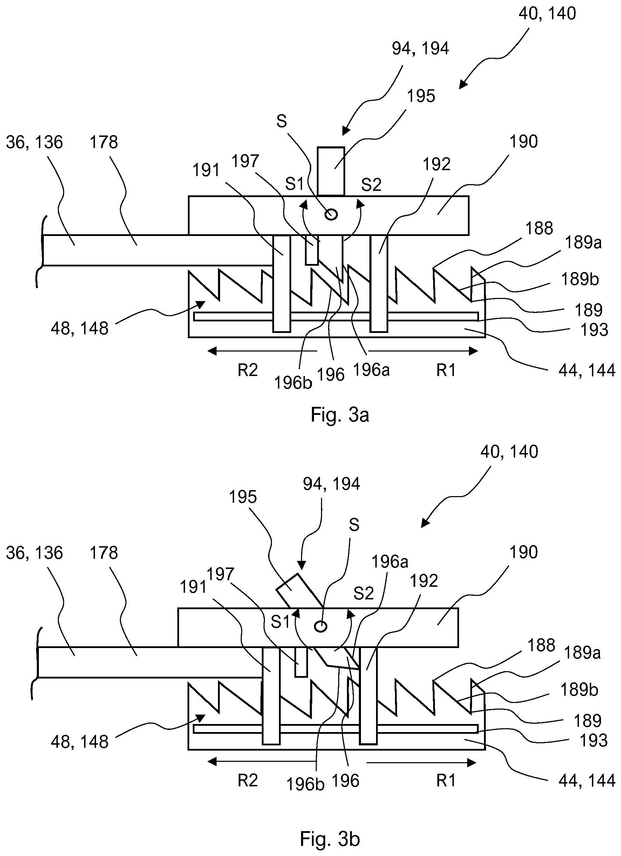

Figuren 3a und 3b zeigen jeweils eine Detaildarstellung der ersten Schienenanordnung 20, 120 mit einer beispielhaften betätigbaren Verstellanordnung 40, 140, dem ersten länglichen Schienenelement 36, 136 und dem zweiten länglichen Schienenelement 44, 144 gemäß der Ausführungsform der Figur 1, wobei die erste Schienenanordnung 120 der Figur 2 im dargestellten Bereich im Wesentlichen gleich ausgebildet ist, sodass die Detaildarstellung auch ein Detail der Schienenanordnung 120 der Figur 2 betreffen könnte.

Das zweite längliche Schienenelement 44, 144 umfasst die Vielzahl an Rastnuten 48, 148, die an diskreten Positionen und gleichmäßig beabstandet an dem zweiten länglichen Schienenelement 44, 144 ausgebildet sind, wobei eine jeweilige Rastnut eine Erhöhung 188 und eine Nut 189 aufweist.

Die Verstellanordnung 40, 140 umfasst ein Verstellteil 190, an dem zwei erste Führungsträger 191 und zwei zweite Führungsträger 192 ausgebildet sind. Der jeweilige, dem Betrachter zugewandte Führungsträger 191, 192 verdeckt den jeweiligen, dem Betrachter abgewandten Führungsträger 191, 192, sodass in der Ansicht der Figur 3 nur die beiden dem Betrachter zugewandten Führungsträger 191, 192 erkennbar sind. Statt der beiden Führungsträger 191, 192 könnte auch ein durchgehender Führungsträger ausgebildet sein, und dabei könnte der Bereich zwischen den Führungsträgern 191, 192 auch als Führungsträger ausgebildet sein. Die Führungsträger 191, 192 weisen nach innen gerichtete Vorsprünge auf (nicht dargestellt), die dazu vorgesehen sind, in einer jeweiligen Führungsnut 193 des zweiten länglichen Schienenelements 44, 144 einzugreifen, sodass die Führungsträger 191, 192 in der jeweiligen Führungsnut 193 geführt sind und damit die Verstellanordnung 40, 140 relativ zu dem zweiten länglichen Schienenelement 44, 144 und damit relativ zu dem ersten Orthesenabschnitt 14, 114 verlagerbar ist. Die dargestellte und die rückseitige Führungsnut 193 erstrecken sich horizontal, parallel und im Wesentlichen entlang der Länge des zweiten länglichen Schienenelements 44, 144. Dadurch, dass die Verstellanordnung 40, 140 zumindest bei Nicht-Betätigung in eine jeweilige Rastnut 48, 148 eingreift, und bei Betätigung ein Ändern der Lage zulässt, wobei die Verstellanordnung 40, 140 zumindest bei erneutem Nicht-Betätigen in dieselbe oder eine andere Rastnut 48, 148 eingreift, so ist ein Ändern der Lage mittels diskreter Positionen zugelassen.

Die Verstellanordnung 40, 140 umfasst ferner die Betätigungsanordnung 94, 194 zum Betätigen der Verstellanordnung 40, 140, wobei die Betätigungsanordnung 94, 194 einen Betätigungsabschnitt 195 zum Betätigen und einen Eingriffsabschnitt 196 umfasst. Die Betätigungsanordnung 94, 194 ist relativ zu dem Verstellteil 190 um die Schwenkachse S verschwenkbar. Der Eingriffsabschnitt 196 ist dazu vorgesehen, in der in Figur 3a gezeigten Eingriffsstellung der Betätigungsanordnung 94, 194 relativ zu dem Verstellteil 190 derart in eine Rastnut 48, 148 einzugreifen, dass ein Ändern der Lage der Verstellanordnung 40, 140 relativ zu dem zweiten länglichen Schienenelement 44, 144 in der ersten Richtung R1 gesperrt ist, wohingegen ein Ändern der Lage der Verstellanordnung 40, 140 relativ zu dem zweiten länglichen Schienenelement 44, 144 in der zweiten Richtung R2 zugelassen ist.

Die Verstellanordnung 40, 140 umfasst einen Anschlag 197, der ein weiteres Verschwenken der Betätigungsanordnung 94, 194 um die Schwenkachse S in einer ersten Schwenkrichtung S1 verhindert. Bei dem Versuch also, die Verstellanordnung 40, 140 relativ zu dem zweiten länglichen Schienenelement 44, 144 in der ersten Richtung R1 zu verlagern, wird der Eingriffsabschnitt 196 mit der Erhöhung 188 in Eingriff gelangen und ein Rotationsmoment in der ersten Schwenkrichtung S1 erfahren, wobei ein weiteres Verschwenken der Betätigungsanordnung 94, 194 in der ersten Schwenkrichtung S1 aufgrund des Anschlags 197 verhindert ist. Die Betätigungsanordnung 94, 194 ist um die Schwenkachse S federvorgespannt, sodass die Betätigungsanordnung 94, 194 relativ zu dem Verstellteil 190 in die Eingriffsstellung der Figur 3a bzw. zur Anlage an dem Anschlag 197 gedrängt wird.

Figur 3b zeigt eine Nichteingriffsstellung des Eingriffsabschnitts 196, wobei die Betätigungsanordnung 94, 194 betätigt ist. Dazu ist die Betätigungsanordnung 94, 194 gegenüber der Eingriffsstellung der Figur 3a um die Schwenkachse S in einer zweiten Schwenkrichtung S2, die der ersten Schwenkrichtung S1 entgegengerichtet ist, so weit verschwenkt, dass der Eingriffsabschnitt 196 außer Eingriff mit den Rastnuten 48, 148, insbesondere den Erhöhungen 188, ist. Somit ist ein Ändern der Lage der Verstellanordnung 40, 140 relativ zu dem zweiten länglichen Schienenelement 44, 144 in der ersten Richtung R1 nicht mehr gesperrt, sondern zugelassen, wobei auch weiterhin ein Ändern der Lage der Verstellanordnung 40, 140 relativ zu dem zweiten länglichen Schienenelement 44, 144 in der zweiten Richtung R2 zugelassen ist.

Wird die Verstellanordnung 40, 140 auch ohne Betätigen der Betätigungsanordnung 94, 194 relativ zu dem zweiten länglichen Schienenelement 44, 144 in der zweiten Richtung R2 verlagert, wird der Eingriffsabschnitt 196 mit der nächsten in Richtung R2 liegenden Erhöhung 188 in Eingriff gelangen, sodass ein Rotationsmoment an dem Eingriffsabschnitt 196 in der zweiten Schwenkrichtung S2 erzeugt wird, sodass der Eingriffsabschnitt 196 beispielsweise in die dargestellte Nichteingriffsstellung schwenkt, zumindest so weit, dass der Eingriffsabschnitt 196 über die Erhöhung 188 gleiten kann und in eine in der Richtung R2 liegende Nut 189 eingreifen kann.

Eine jeweilige Nut 189 umfasst einen im Wesentlichen vertikalen Abschnitt 189a in der ersten Richtung R1 und einen schrägen Abschnitt 189b in der zweiten Richtung R2. Ferner ist der Eingriffsabschnitt 196 mit einem vertikalen Abschnitt 196a und einem schrägen Abschnitt 196b ausgebildet, wobei der vertikale Abschnitt 196a zur Anlage an dem vertikalen Abschnitt 189a vorgesehen ist und der schräge Abschnitt 196b zur Anlage an dem schrägen Abschnitt 189b vorgesehen ist. Alternativ könnte eine jeweilige Nut 189 auch mit zwei im Wesentlichen parallelen, bevorzugt vertikalen Abschnitten vorgesehen sein, und/oder der Eingriffsabschnitt 196 mit zwei vertikalen Abschnitten ausgebildet sein. Auch dann wäre die Verstellanordnung 40, 140 ohne Betätigen der Betätigungsanordnung 94, 194 relativ zu dem zweiten länglichen Schienenelement 44, 144 in der zweiten Richtung R2 verlagerbar, wobei ein Verlagern in der ersten Richtung R1 gesperrt wäre, sofern die Betätigungsanordnung 94, 194 nicht betätigt wird.

Die Figuren 3a und 3b betreffen eine beispielhafte Ausführungsform der Verstellanordnung 40, 140, wobei auch andere Ausführungsformen derselben vorgesehen sein können. Wichtig ist, dass die Verstellanordnung 40, 140 betätigbar ist und bei Betätigung ein translatorisches Ändern der Lage des damit gekoppelten Orthesenabschnitts, vorliegend dem zweiten Orthesenabschnitt 18, 118, relativ zu dem anderen Orthesenabschnitt, vorliegend dem ersten Orthesenabschnitt 14, 114 zugelassen ist, wobei bei Nicht-Betätigung ein translatorisches Ändern der Lage zumindest in einer ersten Richtung gesperrt ist. Statt nur in einer Richtung könnte bei Nicht-Betätigung beispielsweise auch ein translatorisches Ändern der Lage in den beiden Richtungen R1 und R2 gesperrt sein. Man könnte sagen, dass die Verstellanordnung 40, 140 dazu vorgesehen ist, den zweiten Orthesenabschnitt 18, 118 in eine Position relativ zu dem ersten Orthesenabschnitt 14, 114 zu drängen, um eine Traktion auf das Daumensattelgelenk auszuüben.

In den Figuren 3a und 3b ist das erste längliche Schienenelement 36, 136 mit dem Verstellteil 190 gekoppelt, beispielsweise mittels eines Verbindungsmittels wie eines Bolzens, einer Schraube oder einem Niet, wobei auch eine Kleb- oder Schweißverbindung vorgesehen sein könnte. Ferner könnte das erste längliche Schienenelement 36, 136 einteilig mit dem Verstellteil 190 ausgebildet sein. Das erste längliche Schienenelement 36, 136 weist eine im Wesentlichen konstante Dicke und eine im Wesentlichen konstante Breit auf.

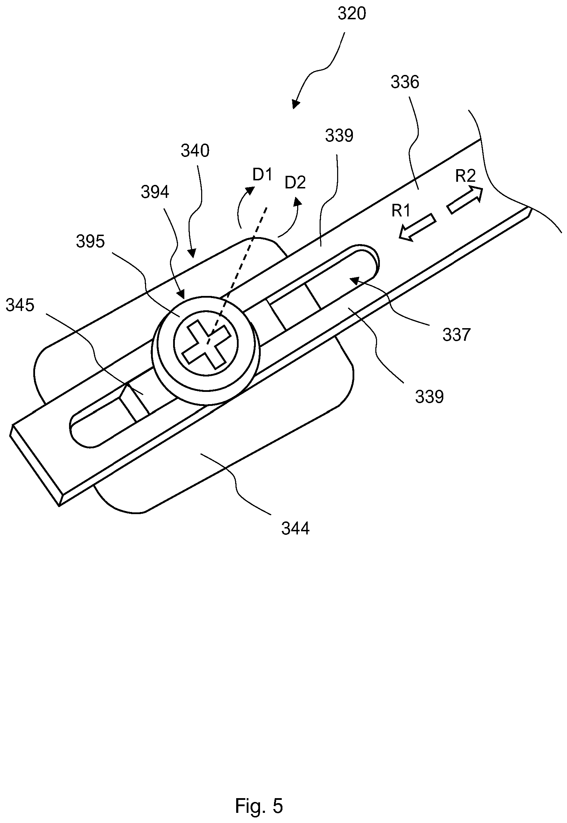

Statt der in den Figuren 3a und 3b gezeigten Ausführungsform könnte die Verstellanordnung 40, 140 auch anders ausgebildet sein und beispielsweise auf einer Klemmwirkung basieren, beispielsweise wie für die Verstellanordnung der Figur 5 erläutert. So könnte das erste längliche Schienenelement 36, 136 in dem unbetätigten Zustand auf den ersten Orthesenabschnitt 14, 114 und/oder das zweite längliche Schienenelement 44, 144 derart gedrückt werden, dass aufgrund der Klemmwirkung ein Ändern der Lage gesperrt ist. Wird die Verstellanordnung 40, 140 hingegen betätigt, so wird die Klemmwirkung zumindest reduziert und ein Ändern der Lage zugelassen. Zum Erzeugen oder Reduzieren der Klemmwirkung könnte beispielsweise eine Schraubverbindung vorgesehen sein, die das erste längliche Schienenelement 36, 136 mit dem zweiten Orthesenabschnitt 14, 114 und/oder dem zweiten länglichen Schienenelement 44, 144 koppelt.

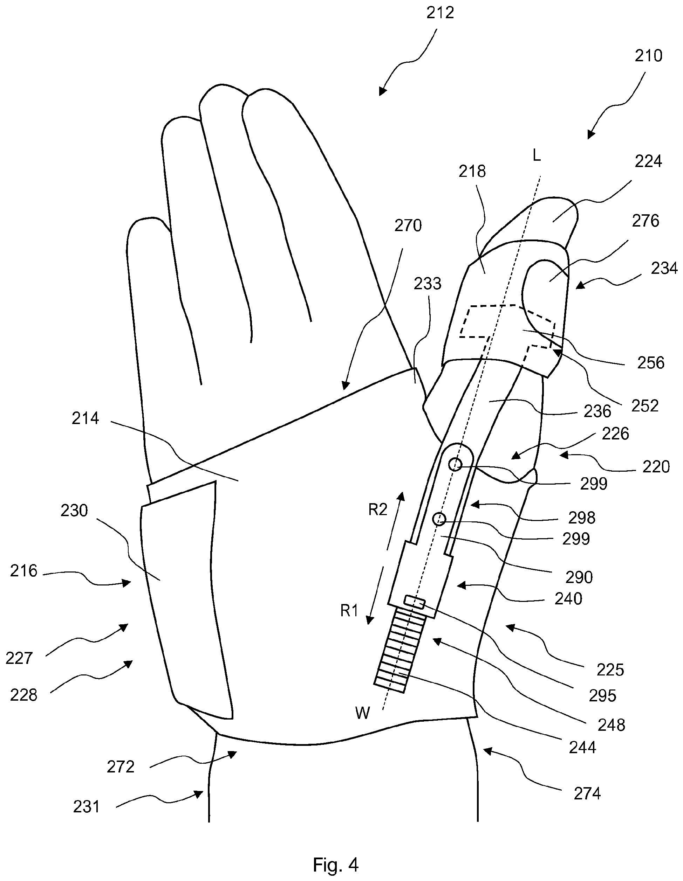

Figur 4 zeigt eine Handorthese 210 zur Traktion eines Fingergelenks gemäß einem dritten Ausführungsbeispiel. Wie auch die Handorthese gemäß Figuren 1 und 2, umfasst die Handorthese 210 einen ersten Orthesenabschnitt 214 zum Anbringen an einem Mittelhandbereich 216 einer Hand 212 des Patienten, einen zweiten Orthesenabschnitt 218 zum Anbringen an einem Finger der Hand 212 des Patienten und eine erste Schienenanordnung 220.

Die Handorthese 210 ist ähnlich wie die Handorthese 10 gemäß Figur 1 und die Handorthese 110 gemäß Figur 2 aufgebaut, wobei im Folgenden besonders auf die Unterschiede eingegangen werden soll. Gleiche o. ä. Merkmal sind mit einem gleichen Bezugszeichen wie in Figur 1 addiert mit 200 dargestellt. Während die Figuren 1 und 2 zumindest teilweise die Handorthese 10, 110 im Bereich eines Handrückens der Hand 12, 112 darstellen, zeigt die Figur 3 die Handorthese zur 210 an der Hand 212 des Patienten im Bereich einer Handinnenfläche der Hand 212. Es versteht sich, dass auch die Handorthesen 10, 110 im Bereich der Handinnenflächen wie die Handorthese 210 ausgebildet sein können, oder die Handorthese 210 im Bereich des Handrückens wie die Handorthesen 10, 110 ausgebildet sein können.

Vorliegend ist die Handorthese 210 beispielhaft als Daumenorthese ausgebildet. Dementsprechend ist der zweite Orthesenabschnitt 218 an einem Daumen 224 der Hand 212 des Patienten angebracht.

Der erste Orthesenabschnitt 214 umfasst eine erste Öffnung 226 zum Einführen bzw. Aufnehmen des Daumens 224 der Hand 212 des Patienten. Diese erste Öffnung 226 umgibt den Daumen 224 an dessen proximalen Ansatz vollständig. Wird die Handorthese 210 an der Hand 212 des Patienten angelegt, dann wird der Daumen 224 durch die erste Öffnung 226 geführt. Der erste Orthesenabschnitt 214 umfasst ferner zumindest eine zweite Öffnung 270 zur Aufnahme der übrigen Finger der Hand 212, also der Finger außer dem Daumen 224. Die zweite Öffnung 270 umgibt einen proximalen Ansatz der übrigen Finger vollständig. Der zweite Orthesenabschnitt 214 ist wie der zweite Orthesenabschnitt 214 der ersten Ausführungsform öffenbar und schließbar ausgebildet. Für dieses optionale Merkmal umfasst der erste Orthesenabschnitt 214 eine weitere Öffnung 227 und einen Verschließmechanismus 228 an einer daumenfernen Seite des ersten Orthesenabschnitts 214. Die weitere Öffnung 227 erstreckt sich vollständig von proximal nach distal durch den ersten Orthesenabschnitt 214. Der Verschließmechanismus 228 umfasst einen Gurt 230, der an dem ersten Orthesenabschnitt 214 im Bereich einer handinnenflächigen Seite des ersten Orthesenabschnitts 214 fest angebracht ist. Beispielsweise kann der Gurt 230 an dem ersten Orthesenabschnitt 214 fest angenäht, geklebt oder mit diesem einteilig ausgebildet sein. Ferner umfasst der Gurt 230 eine nicht dargestellte Klettverschlussanordnung, die mit dem ersten Orthesenabschnitt 214 verbindbar ist, und zwar auf einer dem Handrücken der Hand 212 zuordenbaren Seite des ersten Orthesenabschnitts 214.

Der erste Orthesenabschnitt 214 umfasst ferner eine dritte Öffnung 272 zur Aufnahme eines Handwurzelbereichs 274 der Hand 212 des Patienten. Die dritte Öffnung 272 ist dazu vorgesehen, den Handwurzelbereich 274 der Hand 212 in Umfangsrichtung vollständig zu umgeben.

Wird die Handorthese 210 an der Hand 212 des Patienten angelegt, dann wird zunächst der Verschließmechanismus 228 in einen geöffneten Zustand gebracht, sodass die weitere Öffnung 227 vergrößert oder gänzlich geöffnet ausgebildet ist. Dazu wird der Gurt 230 von der dem Handrücken der Hand 212 zuordenbaren Seite des ersten Orthesenabschnitts 214 gelöst. Anschließend werden alle Finger der Hand 212 durch die dritte Öffnung 272 und/oder die weitere Öffnung 227 geführt. Wiederum anschließend werden der Daumen 224 durch die erste Öffnung 226 und die übrigen Finger durch die zweite Öffnung 270 geführt, bis der erste Orthesenabschnitt 214 an dem Mittelhandbereich 216 anliegt und die dritte Öffnung 272 den Handwurzelbereich 274 in Umfangsrichtung der Hand 212 umgibt. Dann wird die weitere Öffnung 227 mittels des Verschließmechanismus 228 zumindest teilweise geschlossen. Dazu wird der Gurt 230 mit der dem Handrücken der Hand 212 zuordenbaren Seite des ersten Orthesenabschnitts 214 gekoppelt, vorliegend anhand des Klettverschlusses. Dabei wird die Form des ersten Orthesenabschnitts 214 an die Form des Mittelhandbereichs 216 angepasst. Der erste Orthesenabschnitt 214 umgibt den Mittelhandbereich 216 in Umfangsrichtung in dem angelegten Zustand vollständig.

Der erste Orthesenabschnitt 214 bzw. eine räumliche Erstreckung desselben ist auf den Mittelhandbereich 216 beschränkt. Dies bedeutet, dass der erste Orthesenabschnitt 214 einen Handgelenkbereich 231, den Daumen 224 und/oder andere Finger der Hand 212 und/oder Fingergrundgelenke dieser anderen Finger der Hand 212 nicht überlagert. Damit wird eine besonders schlanke Form einer Handorthese 210 bereitgestellt, die die Arm-, Hand- und Fingerbewegungen, abgesehen von der Bewegung des Daumens, nicht oder lediglich kaum einschränkt.

Der erste Orthesenabschnitt 214 umfasst auch einen nicht dargestellten, dem Handrücken der Hand 212 zuordenbaren Bereich, an dem vorliegend der Gurt 230 anbringbar ist. Der Gurt 230 erstreckt sich somit über eine Handunterseite der Hand 212 zu dem Bereich des Handrückens der Hand 212. Der erste Orthesenabschnitt 212 umfasst einen Übergangsabschnitt 233, der zwischen der ersten Öffnung 226 und der zweiten Öffnung 270 ausgebildet ist, und dazu vorgesehen ist, in dem an der Hand 212 angeordneten Zustand der Handorthese 210 zwischen dem Daumen 224 und den übrigen Fingern der Hand 212 angeordnet zu sein. Der Übergangsabschnitt 233 verbindet den dargestellten handinnenseitigen Bereich des ersten Orthesenabschnitts 214 mit dem nicht dargestellten, handrückenseitigen Bereich des ersten Orthesenabschnitts 214 im Bereich zwischen dem Daumen 224 und den übrigen Fingern der Hand 212.

Der zweite Orthesenabschnitt 218 ist ringförmig ausgebildet und umgibt den Daumen 224 in Umfangsrichtung vollständig und auch proximal des Daumenendgelenks 234 der Hand 212. Man könnte auch sagen, dass der zweite Orthesenabschnitt 218 zumindest zum Anliegen an den proximalen Kondylen des Daumenendgelenks 234 vorgesehen ist, um auf diese eine nach distal gerichtete Kraft zum Erzeugen der Traktion auf ein Daumensattelgelenk 225 (nicht dargestellt) des Daumens 224 auszuüben. Der zweite Orthesenabschnitt 218 ist getrennt von und beabstandet zu dem ersten Orthesenabschnitt 214 ausgebildet.

Der zweite Orthesenabschnitt 218 ist zumindest bis zu einem gewissen Grad elastisch ausgebildet, sodass der zweite Orthesenabschnitt 18 zum Anlegen der Handorthese 210 über den Daumen 24 gezogen werden kann, er sich dabei weitet und anschließend an die Daumenform angepasst proximal des Daumenendgelenks 234 an dem Daumenendgelenk 234 anliegt.

Der zweite Orthesenabschnitt 218 ist öffenbar und verschließbar ausgebildet. Dies ermöglicht es, den zweiten Orthesenabschnitt 218 einfach an dem Daumen 224 anzubringen und an die tatsächliche Größe des Daumens 224 anzupassen. Zum Öffnen und Schließen umfasst der zweite Orthesenabschnitt 218 eine Lasche 276, wobei der zweite Orthesenabschnitt 218 beim Anlegen desselben in Umfangsrichtung um den Daumen 224 gelegt und die Lasche 276 mit einer Außenseite des zweiten Orthesenabschnitts 218 je nach Daumengröße oder Patientenbelieben gekoppelt werden kann. Die Lasche 276 umfasst dazu eine Art Klettverschluss, der mit der Außenseite des zweiten Orthesenabschnitts 218 koppelbar ist.

Die erste Schienenanordnung 220 umfasst ein erstes längliches Schienenelement 236, das mit dem zweiten Orthesenabschnitt 218 gekoppelt ist. Ferner umfasst die Schienenanordnungen 20 eine betätigbare Verstellanordnung 240, die im Wesentlichen wie in den Figuren 3a und 3b gezeigt ausgebildet ist bzw. auf dem in den Figuren 3a und 3b gezeigten Funktionsprinzip basiert. Die Verstellanordnung 240 ist dazu eingerichtet, bei Betätigung ein Ändern der Lage des ersten länglichen Schienenelements 236 und dem damit gekoppelten zweiten Orthesenabschnitt 218 relativ zu dem ersten Orthesenabschnitt 214 zuzulassen. Dazu umfasst die Schienenanordnung 220 in der vorliegenden Ausführungsform ein zweites längliches Schienenelement 244, das mit dem ersten Orthesenabschnitt 214 gekoppelt ist. Man könnte auch sagen, dass das zweite längliche Schienenelement 244 an dem ersten Orthesenabschnitt 214 fest angebracht ist. Das zweite längliche Schienenelement 244 umfasst eine Mehrzahl an Rastnuten 248, in die die Verstellanordnung 240 eingreifen kann. Die Verstellanordnung 240 ist mit dem ersten länglichen Schienenelement 236 gekoppelt. Wie auch in den Figuren 3a und 3b dargestellt, umfasst die Verstellanordnung 240 ein Verstellteil 290, das allerdings größer ausgebildet ist und das erste längliche Schienenelement 236 in einem Überlappungsbereich 298 überlappt. Ferner sind zwei Befestigungsmittel 299 vorgesehen, die vorliegend als jeweilige Niete ausgebildet sind, die die Verstellanordnung 240 mit dem ersten länglichen Schienenelement 236 koppeln. Der groß gewählte Überlappungsbereich 298 bewirkt eine erhöhte Stabilität und verbesserte Kraftübertragung zwischen der Verstellanordnung 240 und dem ersten länglichen Schienenelement 236.

Die Verstellanordnung 240 umfasst ferner einen Betätigungsabschnitt 295 zum Betätigen der Verstellanordnung 240, wie er im Zusammenhand mit den Figuren 3a und 3b erläutert ist, und einen nicht dargestellten Eingriffsabschnitt, wie er ebenfalls im Zusammenhand mit den Figuren 3a und 3b erläutert ist. Die Verstellanordnung 240 ist relativ zu und an dem zweiten länglichen Schienenelement 244 verschieblich gelagert. Ist die Verstellanordnung 240 nicht betätigt, so rastet der Eingriffsabschnitt der Verstellanordnung 240 in einer der Rastnuten 248 ein, sodass ein Ändern der Lage der Verstellanordnung 240 nicht oder zumindest nur in einer zweiten Richtung R1 zugelassen ist, wohingegen ein Ändern der Lage der Verstellanordnung 240 in einer ersten Richtung R2 relativ zu dem zweiten länglichen Schienenelement 244 nicht zugelassen ist. Ist der Betätigungsabschnitt 295 der Verstellanordnung 240 betätigt, so rastet der Eingriffsabschnitt der Verstellanordnung 240 nicht in eine der Rastnuten 248 ein. Somit ist ein Ändern der Lage der Verstellanordnung 240 relativ zu dem jeweiligen zweiten länglichen Schienenelement 244 zugelassen, gemäß der vorliegenden Ausführungsform in der ersten Richtung R1 und in der zweiten Richtung R2. Dieses Prinzip ähnelt einem betätigbaren Kabelbinderverschluss. Die zweite Richtung R2 ist entgegengesetzt zu der ersten Richtung R1 ausgerichtet. Die Lage des zweiten Orthesenabschnitts 218 relativ zu dem ersten Orthesenabschnitt 214 kann vorgegeben werden, indem die Lage wie erläutert verändert wird und die Verstellanordnung 240 in eine gewünschte der Rastnuten 248 eingreift. Die Vorgabe der Lage beeinflusst die Traktion auf das Fingergelenk, vorliegend das Daumensattelgelenk.

Eine Längsachse W des zweiten länglichen Schienenelements 244, entlang derer die Lage der Verstellanordnung 240 relativ zu dem zweiten länglichen Schienenelement 244 geändert werden kann, ist im Wesentlichen kongruent und/oder parallel zu einer Daumenlängsachse L des Daumens 224 ausgerichtet, wenn der Daumen 224 in einer im Wesentlichen gestreckten Position - wie dargestellt - ausgerichtet ist.

Das erste längliche Schienenelement 236 ist länglich ausgebildet und an die Form des Daumens 224 angepasst. Der zweite Orthesenabschnitt 218 umfasst eine erste Tasche 252 zur Aufnahme eines ersten Endes 256 des ersten länglichen Schienenelements 236. Das Ende 256 ist flächig ausgebildet, um ein flächiges Anliegen an dem Daumen 224 im Bereich des zweiten Orthesenabschnitts 218 bereitzustellen. Vorliegenden ist das Ende 256 T-förmig ausgebildet, könnten aber auch P-, D-, O-förmig oder ähnlich oder halbkreisförmig den Daumen 224 in Umfangsrichtung im Wesentlichen hälftig oder vollständig umfassend ausgebildet sein. Statt der ersten Tasche 252 könnte das jeweilige Ende 256 auch lediglich daumenseitig zu dem zweiten Orthesenabschnitt 218 angeordnet sein und von dem zweiten Orthesenabschnitt 218 an den Finger geklemmt werden. Alternativ könnte das Ende 256 auch auf eine andere Art mit dem zweiten Orthesenabschnitt 218 gekoppelt sein, beispielsweise geklebt, verschweißt, genietet etc.

Das Ausmaß der Traktion kann wie vorangehend zu den Figuren 1, 2, 3a und 3b angepasst werden.

Zusätzlich zu der ersten Schienenanordnung 220 könnte an der Handorthese 210 gemäß der dritten Ausführungsform auch eine zweite Schienenanordnung ausgebildet sein. Eine solche zweite Schienenanordnung könnte wie in den Ausführungsformen der Figuren 1 oder 2 ausgebildet und/oder angeordnet sein. Ferner könnte eine solche zweite Schienenanordnung wie die erste Schienenanordnung 220 ausgebildet sein, aber lediglich an dem ersten Orthesenabschnitt 214 und/oder zweiten Orthesenabschnitt 218 bevorzugt an dem handrückseitigen und nicht dargestellten Bereich angeordnet sein, beispielsweise wie in den Figuren 1 oder 2 dargestellt. Es versteht sich, dass auch drei, vier oder mehr solcher Schienenanordnungen vorgesehen sein könnten.

Es versteht sich, dass einzelne oder mehrere Bauteile und/oder Merkmale der verschiedenen Ausführungsformen der Handorthese 10, 110, 210 mit einer jeweiligen anderen dargestellten Ausführungsform der Handorthese 10, 110, 210 kombiniert werden können. Beispielsweise könnten der jeweilige erste Orthesenabschnitt 14, 114, 214 und/oder der jeweilige zweite Orthesenabschnitt 18, 118, 218 an einer der anderen Ausführungsformen der Handorthese vorgesehen sein.

Figur 5 zeigt eine schematische Ansicht einer Schienenanordnung 320 gemäß einem weiteren Ausführungsbeispiel, wobei die Schienenanordnung 320 in den vorangehend beschriebenen Ausführungsformen der Handorthesen 10, 110, 210 statt oder zusätzlich zu den dort beschriebenen Schienenanordnungen 20, 22, 120, 122 vorgesehen sein kann. Die Schienenanordnung 320 umfasst das erste längliche Schienenelement 336, das mit dem nicht dargestellten zweiten Orthesenabschnitt gekoppelt ist, das zweite längliches Schienenelement 344, das mit dem nicht dargestellten ersten Orthesenabschnitt gekoppelt ist, und eine betätigbare Verstellanordnung 340 gemäß dem Ausführungsbeispiel. Die Verstellanordnung 340 umfasst eine Schraubverbindung und basiert auf einer Klemmwirkung der Schraubverbindung. Man könnte sagen, die Verstellanordnung 340 umfasst eine Klemmanordnung.

Das zweite längliche Schienenelement 344 umfasst eine im Wesentlichen rechteckförmige Platte, wobei dieses auch andere Formen aufweisen könnte und/oder durch einen der Orthesenabschnitte, insbesondere den ersten Orthesenabschnitt, ausgebildet sein kann.

Die Verstellanordnung 340 ist an dem zweiten länglichen Schienenelement 344 angebracht und umfasst eine Betätigungsanordnung 394, die drehbar relativ zu und/oder in dem zweiten länglichen Schienenelement 344 gelagert ist. Dazu ist die Betätigungsanordnung 394 als eine Art Schraube ausgebildet, die mit dem zweiten länglichen Schienenelement 344 in Gewindeeingriff steht, sodass eine Bewegung der Betätigungsanordnung 394 in einer ersten Drehrichtung D1 relativ zu dem zweiten länglichen Schienenelement 344 zu einer Befestigungsbewegung und eine Bewegung der Betätigungsanordnung 394 in einer zweiten, entgegengesetzten Drehrichtung D2 relativ zu dem zweiten länglichen Schienenelement 344 zu einer Lockerungsbewegung führt. Ferner ist ein optionaler Führungskörper 345 zwischen einem als Betätigungskopf ausgebildeten Betätigungsabschnitt 395 der Betätigungsanordnung 394 und dem zweiten länglichen Schienenelement 344 angeordnet. Man könnte sagen, dass ein Gewinde bzw. ein Schraubenschaft der Betätigungsanordnung 394 durch den Führungskörper 345 und in das zweite längliche Schienenelement 344 ragt.

Das erste längliche Schienenelement 336 umfasst eine längliche Aussparung 337 zur gleitenden Führung des Führungskörpers 345. Ferner ragt das Gewinde bzw. der Schraubenschaft (nicht dargestellt) der Betätigungsanordnung 394 durch die Aussparung 337 in das zweite längliche Schienenelement 344. Die Aussparung 337 weist eine Länge auf, die ein Vielfaches einer Breite der Aussparung 337 beträgt. Ein Durchmesser des Betätigungsabschnitts 395 ist größer ausgebildet als die Breite der Aussparung 337. Man könnte auch sagen, dass im Bereich der Aussparung 337 zwei gegenüberliegende und parallel zueinander ausgerichtete Stege 339 ausgebildet sind, die dazu vorgesehen sind, den Führungskörper 345 zu führen. Die Stege 339 sind zwischen dem zweiten länglichen Schienenelement 344 und dem Betätigungsabschnitt 395 angeordnet.

Die Verstellanordnung 340 kann betätigt werden. Dazu kann ein Benutzer wie ein Patient oder Therapeut den Betätigungsabschnitt 395 in der Drehrichtung D2 drehen. Dadurch wird das erste längliche Schienenelement 336, insbesondere die Stege 339, nicht mehr von der Verstellanordnung 340 und dem zweiten länglichen Schienenelement 344 geklemmt, sodass das erste längliche Schienenelement 336 relativ zu dem zweiten länglichen Schienenelement 344 und der Verstellanordnung 340 in der ersten und zweiten Richtung R1, R2 verlagerbar ist. Dadurch kann der Bediener das erste längliche Schienenelement 336 in eine gewünschte Lage bringen und die beiden Orthesenabschnitte (nicht gezeigt), die mit einem jeweilen der länglichen Schienenelemente 344, 336 gekoppelt sind, relativ zueinander in einer vorgebbaren Lage anordnen. Anschließend wird der Betätigungsabschnitt 395 in der Drehrichtung D1 bewegt, bis das erste längliche Schienenelement 336 erneut zwischen dem zweiten länglichen Schienenelement 344 und der Verstellanordnung 340 geklemmt wird, sodass das erste längliche Schienenelement 336 nicht relativ zu dem zweiten länglichen Schienenelement 344 verlagerbar ist. Somit ist die Lage des ersten länglichen Schienenelements 336 und dem damit gekoppelten Orthesenabschnitt relativ zu dem anderen Orthesenabschnitt erneut fest und ein Ändern der Lage gesperrt.

Der Betätigungsabschnitt 395 kann an dessen Oberfläche ein geometrisches Merkmal wie eine Riffelung oder ähnliches aufweisen, das einem Patienten ein besseres Greifen und Betätigen ermöglicht. Das Gewinde bzw. der Schaft der Verstellanordnung kann auch fest an dem zweiten länglichen Schienenelement 344 ausgebildet sein, und die Betätigungsanordnung 394 als eine Art Schraubenmutter ausgebildet sein, die mit dem Gewinde in Eingriff steht. Dabei kann der Betätigungsabschnitt 395 auch beispielsweise als Gewindemutter, insbesondere Flügelmutter, ausgebildet sein. Ferner kann an dem Betätigungsabschnitt 395 ein geometrisches Merkmal für einen Eingriff mit einem Werkzeug ausgebildet sein. Ein solches geometrisches Merkmal kann ein Innensechskantprofil, Außensechskantprofil, Schlitzprofil, Kreuzschlitzprofil oder ähnliches umfassen.

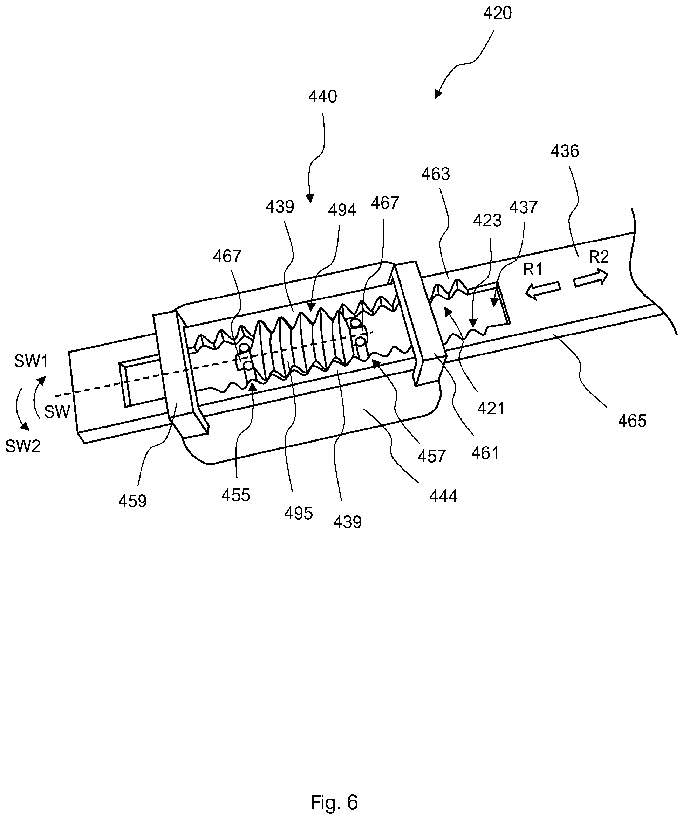

Figur 6 zeigt eine schematische Ansicht einer Schienenanordnung 420 gemäß einem wiederum weiteren Ausführungsbeispiel, wobei die Schienenanordnung 420 in den vorangehend beschriebenen Ausführungsformen der Handorthesen 10, 110, 210 statt oder zusätzlich zu den dort beschriebenen Schienenanordnungen 20, 22, 120, 220 vorgesehen sein kann. Die Schienenanordnung 420 umfasst das erste längliche Schienenelement 436, das mit dem nicht dargestellten zweiten Orthesenabschnitt gekoppelt ist, das zweite längliche Schienenelement 444, das mit dem nicht dargestellten ersten Orthesenabschnitt gekoppelt ist, und eine betätigbare Verstellanordnung 440. Die Verstellanordnung 440 umfasst ein Schneckengetriebe.

Das zweite längliche Schienenelement 444 umfasst eine im Wesentlichen rechteckförmige Platte, wobei dieses auch andere Formen aufweisen könnte und/oder durch einen der Orthesenabschnitte, insbesondere den ersten Orthesenabschnitt, ausgebildet sein kann.

Die Verstellanordnung 440 ist einerseits an dem zweiten länglichen Schienenelement 444 ausgebildet und umfasst eine Betätigungsanordnung 494, die drehbar relativ zu und an dem zweiten länglichen Schienenelement 444 gelagert ist. Dazu ist die Betätigungsanordnung 494 als eine Art Schneckenwelle 495 ausgebildet, die mit dem ersten länglichen Schienenelement 436 in Gewindeeingriff bzw. zahnendem Eingriff steht, sodass eine Rotation der Betätigungsanordnung 494 in einer ersten Schneckenwellendrehrichtung SW1 um eine Schneckenwellenlängsachse SW relativ zu dem zweiten länglichen Schienenelement 444 zu einer Relativbewegung von dem ersten länglichen Schienenelement 436 und dem zweiten länglichen Schienenelement 444 führt, bei der vorliegend das erste längliche Schienenelement 436 in der ersten Richtung R1 relativ zu dem zweiten länglichen Schienenelement 444 verlagert wird. Ferner führt eine Rotation der Betätigungsanordnung 494 in einer zweiten Schneckenwellendrehrichtung SW2 um die Schneckenwellenlängsachse SW relativ zu dem zweiten länglichen Schienenelement 444 zu einer entgegengesetzten Relativbewegung von dem ersten länglichen Schienenelement 436 und dem zweiten länglichen Schienenelement 444 führt, bei der vorliegend das erste längliche Schienenelement 436 in der zweiten Richtung R2 relativ zu dem zweiten länglichen Schienenelement 444 verlagert wird. Die Richtungen R1 und R2 fallen mit der Schneckenwellenachse SW zusammen.

Für den Gewindeeingriff umfasst das erste längliche Schienenelement 436 eine längliche Aussparung 437 zur Aufnahme der Betätigungsanordnung 494. Die Aussparung 337 weist eine Länge auf, die ein Vielfaches einer Breite der Aussparung 337 beträgt. Man könnte auch sagen, dass im Bereich der Aussparung 337 zwei gegenüberliegende Stege 439 ausgebildet sind, die dazu vorgesehen sind, von der Betätigungsanordnung 445 geführt zu werden. Dazu ist an dem ersten Steg 439 ein erster Zahnabschnitt 421 mit einer Vielzahl an Zähnen und an dem zweiten Steg 439 ein zweiter Zahnabschnitt 423 ausgebildet, wobei die Zahnabschnitte 421, 423 teilweise mit dem Gewinde der Schneckenwelle 495 in Eingriff stehen und zum Eingriff mit dem Gewinde der Schneckenwelle 495 vorgesehen sind. Dadurch, dass die Zähne an dem ersten länglichen Schienenelement 436 ausgebildet sind, könnte man auch sagen, dass die Verstellanordnung 440, insbesondere das Schneckengetriebe, zumindest teilweise an dem ersten länglichen Schienenelement 436 ausgebildet ist.

An dem zweiten länglichen Schienenelement 444 ist eine erste Lageranordnung 455 und eine zweite Lageranordnung 457 vorgesehen, die jeweilige Enden der Schneckenwelle 495 drehbar um die Schneckenwellenlängsachse SW lagern. Dazu sind an den Enden der Schneckenwelle 495 zylindrische Führungszapfen 467 ausgebildet, wobei die jeweilige Zylinderlängsachse der Führungszapfen 467 mit der Schneckenwellenlängsachse SW zusammenfällt, und die Führungszapfen 467 drehbar in der jeweiligen Lageranordnung 455, 457 gelagert ist.

Ferner sind an dem zweiten länglichen Schienenelement 444 zumindest ein erster Führungsbogen 459 und ein zweiter Führungsbogen 461 ausgebildet, die jeweils dazu vorgesehen sind, das erste längliche Schienenelement 436 relativ zu dem zweiten länglichen Schienenelement 444 entlang der beiden Richtungen R1 und R2 gleitend zu führen. Dazu ist das erste längliche Schienenelement 436 mit einer dem zweiten länglichen Schienenelement 444 zugewandten Seite, vorliegend einer Unterseite, mit diesem in Kontakt. Ferner ist eine Oberseite 463 des ersten länglichen Schienenelements 436 mit den Führungsbögen 459, 461 in Kontakt, wobei ferner die zwei lateralen Seiten 465 des ersten länglichen Schienenelements 436 ebenfalls mit den Führungsbögen 459, 461 in Kontakt stehen. Die Führungsbögen 459, 461 sind U-förmig ausgebildet, wobei eine innere Höhe der U-Form im Wesentlichen der Höhe des ersten länglichen Schienenelements 436 und eine Breite der U-Form im Wesentlichen der Breite des ersten länglichen Schienenelements 436 entspricht, wobei eine gleitende, im Wesentlichen spielfreie Lagerung bereitgestellt ist.

Durch das Betätigen der Verstellanordnung 440 kann der Bediener das erste längliche Schienenelement 436 in eine gewünschte Lage relativ zu dem zweiten länglichen Schienenelement 444 bringen und die beiden Orthesenabschnitte (nicht gezeigt), die jeweils mit einem der länglichen Schienenelemente 444, 436 gekoppelt sind, relativ zueinander in einer vorgebbaren Lage anordnen.

Ein Vorteil der Ausbildung der Verstellanordnung 440 mit der Schneckenwelle 495 ist, dass die Schneckenwelle 495 einfach durch einen Bediener betätigt werden kann, indem die Schneckenwelle 495 beispielsweise mit einem Finger des Bedieners in einer der Schneckenwellendrehrichtungen SW1, SW2 gedreht werden, woraufhin eine translatorische Verlagerung des ersten länglichen Schienenelements 436 in einer der Richtungen R1, R2 und in Richtung der Schneckenwellendrehachse SW bewirkt wird. Ein weiterer Vorteil ist die selbsthemmende Eigenschaft der Verstellanordnung 440. Dies bedeutet, dass eine Kraft auf das erste längliche Schienenelement 436 in einer der Richtungen R1, R2 nicht zu einer Bewegung der Schneckenwelle 495 in einer der Schneckenwellendrehrichtungen SW1, SW2 führt. Ein wiederum weiterer Vorteil ist die schlanke Ausführung der Verstellanordnung 440.

## Patentansprüche

1. Handorthese (10; 110; 210) zur Traktion eines Fingergelenks, insbesondere eines Daumensattelgelenks (25; 225), eines Patienten, umfassend
einen ersten Orthesenabschnitt (14; 114; 214) zum Anbringen an einem Mittelhandbereich (16; 116; 216) einer Hand (12; 112; 212) des Patienten, wobei der erste Orthesenabschnitt (14; 114; 214) zumindest eine erste Öffnung (26; 126; 226) zum Einführen eines Fingers der Hand (12; 112; 212) des Patienten umfasst;
einen zweiten Orthesenabschnitt (18; 118; 218) zum Anbringen an dem Finger der Hand (12; 112; 212) des Patienten; und
zumindest eine Schienenanordnung (20, 22; 120; 220; 320; 420), die den ersten Orthesenabschnitt (14; 114; 214) und den zweiten Orthesenabschnitt (18; 118; 218) aneinanderkoppelt;
**dadurch gekennzeichnet, dass** die Schienenanordnung (20, 22; 120; 220; 320; 420) dazu vorgesehen ist, den zweiten Orthesenabschnitt (18; 118; 218) in einer vorgebbaren Lage relativ zu dem ersten Orthesenabschnitt (14; 114; 214) anzuordnen.

2. Handorthese (10; 110; 210) nach einem der vorangehenden Ansprüche, **dadurch gekennzeichnet, dass** die Schienenanordnung (20, 22; 120; 220; 320; 420)
- zumindest ein erstes längliches Schienenelement (36, 38; 136; 236; 336; 436), das mit einem von dem ersten Orthesenabschnitt (14; 114; 214) und dem zweiten Orthesenabschnitt (18; 118; 218) gekoppelt ist; und
- eine betätigbare Verstellanordnung (40, 42; 140; 240; 340; 440) umfasst;
wobei die Verstellanordnung (40, 42; 140; 240; 340; 440) dazu eingerichtet ist, zumindest bei Betätigung ein Ändern, insbesondere translatorisches Ändern, der Lage des ersten länglichen Schienenelements (36, 38; 136; 236; 336; 436) und dem damit gekoppelten Orthesenabschnitt (14, 114, 214; 18, 118, 218) relativ zu dem anderen von dem ersten und dem zweiten Orthesenabschnitt (14, 114, 214; 18, 118, 218) zuzulassen und/oder zu bewirken.

3. Handorthese (10; 110; 210) nach Anspruch 2, **dadurch gekennzeichnet, dass** die Verstellanordnung (40, 42; 140; 240; 340; 440) dazu vorgesehen ist, bei Betätigung ein Ändern der Lage mittels diskreter Positionen zuzulassen.

4. Handorthese (10; 110; 210) nach Anspruch 2 oder 3, **dadurch gekennzeichnet, dass** die Verstellanordnung (40, 42; 140; 240; 340; 440) dazu eingerichtet ist, bei Nicht-Betätigung ein Ändern der Lage zumindest in einer ersten Richtung (R1) zu sperren und bei Betätigung ein Ändern der Lage zumindest in der ersten Richtung (R1) freizugeben.

5. Handorthese (10; 110; 210) nach einem der Ansprüche 2 bis 4, **dadurch gekennzeichnet, dass** die Verstellanordnung (40, 42; 140; 240; 340; 440) ferner dazu eingerichtet ist, ein Ändern der Lage in einer zweiten Richtung (R2) sowohl bei einer Nicht-Betätigung als auch bei einer Betätigung der Verstellanordnung (40, 42; 140; 240; 340; 440) zuzulassen, wobei die zweite Richtung (R2) bevorzugt zu der ersten Richtung (R1) entgegengesetzt ausgerichtet ist.

6. Handorthese (10; 110; 210) nach einem der Ansprüche 2 bis 4, **dadurch gekennzeichnet, dass** die Verstellanordnung (40, 42; 140; 240; 340; 440) ein Schneckengetriebe umfasst, das dazu vorgesehen ist, bei Betätigung ein Ändern, insbesondere translatorisches Ändern, der Lage des ersten länglichen Schienenelements (36, 38; 136; 236; 336; 436) und dem damit gekoppelten Orthesenabschnitt (14, 114, 214; 18, 118, 218) relativ zu dem anderen von dem ersten und dem zweiten Orthesenabschnitt (14, 114, 214; 18, 118, 218) zu bewirken.

7. Handorthese (10; 110; 210) nach einem der vorangehenden Ansprüche, **dadurch gekennzeichnet, dass** das erste längliche Schienenelement (36, 38; 136; 236; 336; 436) Y-förmig ausgebildet ist.

8. Handorthese (10; 110; 210) nach einem der vorangehenden Ansprüche, **dadurch gekennzeichnet, dass** die Schienenanordnung (20, 22; 120; 220; 320; 420) ferner ein zweites längliches Schienenelement (44, 46; 144; 244; 344; 444) umfasst, das mit dem anderen von dem ersten und dem zweiten Orthesenabschnitt (14, 114, 214; 18, 118, 218) gekoppelt ist.

9. Handorthese (10; 110; 210) nach Anspruch 8, **dadurch gekennzeichnet, dass** die Verstellanordnung (40, 42; 140; 240; 340; 440) dazu eingerichtet ist, bei Betätigung ein Ändern, insbesondere translatorisches Ändern, der Lage des zweiten länglichen Schienenelements (44, 46; 144; 244; 344; 444) und dem damit gekoppelten ersten oder zweiten Orthesenabschnitt (14, 114, 214; 18, 118, 218) relativ zu dem ersten länglichen Schienenelement (36, 38; 136; 236; 336; 436) zuzulassen.

10. Handorthese (10; 110; 210) nach einem der vorangehenden Ansprüche, **dadurch gekennzeichnet, dass** die Schienenanordnung (20, 22; 120; 220; 320; 420) im Wesentlichen biegesteif und/oder torsionssteif ausgebildet ist.

11. Handorthese (10; 110; 210) nach einem der vorangehenden Ansprüche, **dadurch gekennzeichnet, dass** eine räumliche Erstreckung des ersten Orthesenabschnitts (14; 114; 214) in einem an der Hand (12; 112; 212) des Patienten angebrachten Zustand im Wesentlichen auf den Mittelhandbereich (16; 116; 216) beschränkt ist.

12. Handorthese (10; 110; 210) nach einem der vorangehenden Ansprüche, **dadurch gekennzeichnet, dass** der zweite Orthesenabschnitt (18; 118; 218) dazu eingerichtet ist, positionstreu relativ zu dem Finger angebracht zu werden und/oder der erste Orthesenabschnitt (14; 114; 214) dazu eingerichtet ist, positionstreu relativ zu dem Mittelhandbereich (16; 116; 216) angebracht zu werden.

13. Handorthese (10; 110; 210) nach einem der vorangehenden Ansprüche, **dadurch gekennzeichnet, dass** der zweite Orthesenabschnitt (18; 118; 218) in dem an dem Finger angebrachten Zustand nur einen Teil des Fingers in einer Fingerlängsrichtung umgreift.

14. Handorthese (10; 110; 210) nach einem der vorangehenden Ansprüche, **dadurch gekennzeichnet, dass** der erste Orthesenabschnitt (14; 114; 214) und/oder der zweite Orthesenabschnitt (18; 118; 218) öffenbar ausgebildet ist.

15. Handorthese (10; 110; 210) nach einem der vorangehenden Ansprüche, **gekennzeichnet durch** eine zweite Schienenanordnung (22), die den ersten Orthesenabschnitt (14; 114; 214) und den zweiten Orthesenabschnitt (18; 118; 218) aneinanderkoppelt und dazu vorgesehen ist, den zweiten Orthesenabschnitt (18; 118; 218) in einer vorgebbaren Lage relativ zu dem ersten Orthesenabschnitt (14; 114; 214) anzuordnen.
